# EUROPÄISCHE PATENTSCHRIFT

(11) **EP 2 705 552 B1**
(45) Veröffentlichungstag und Bekanntmachung des Hinweises auf die Patenterteilung: **04.03.2015**
(21) Anmeldenummer: 12721754.5
(22) Anmeldetag: 14.04.2012
(51) Int. Cl.: H01L 51/30, C07D 219/12

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 05.05.2011 EP 11003705; 31.08.2011 EP 11007067
(43) Veröffentlichungstag der Anmeldung: 12.03.2014
(73) Patentinhaber: Merck Patent GmbH, 64293 Darmstadt (DE)
(72) Erfinder: MONTENEGRO, Elvira, 69469 Weinheim (DE); BUESING, Arne, 65929 Frankfurt am Main (DE); VOGES, Frank, 67098 Bad Duerkheim (DE); PFLUMM, Christof, 60291 Darmestadt (DE)
(86) Internationale Anmeldenummer: PCT/EP2012/001624
(87) Internationale Veröffentlichungsnummer: WO 2012/150001

(56) Entgegenhaltungen:
- US-A1- 2008 008 907
- US-A1- 2010 171 417

## Beschreibung

Die vorliegende Erfindung betrifft eine Verbindung der Formel (I), die Verwendung der Verbindung in einer elektronischen Vorrichtung, sowie eine elektronische Vorrichtung enthaltend eine Verbindung der Formel (I). Weiterhin betrifft die vorliegende Erfindung ein Verfahren zur Herstellung einer Verbindung der Formel (I) sowie eine Formulierung enthaltend eine oder mehrere Verbindungen der Formel (I).

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik ist die Verwendung insbesondere von Arylaminverbindungen und Carbazolverbindungen als Lochtransportmaterialien für OLEDs bekannt.

Die Anmeldung WO 2010/083871 offenbart die Verwendung von Dihydroacridin-Derivaten, welche mit einer oder mehreren Arylaminogruppen substituiert sind, als Funktionsmaterialien in OLEDs, bevorzugt als Lochtransport- und Lochinjektionsmaterialien.

Weiterhin offenbart die Anmeldung WO 2011/107186 die Verwendung von Dihydroacridin-Derivaten, welche mit einer oder mehreren Carbazolgruppen substituiert sind, als Funktionsmaterialien in OLEDs, bevorzugt als Lochtransport- und Lochinjektionsmaterialien.

Nochmals weiterhin offenbart die Anmeldung US 2010/0019658 die Verwendung von Dihydroacridin-Derivaten, welche Aryl- oder Heteroarylgruppen als Substituenten der Methylengruppe des Dihydroacridins tragen, als Funktionsmaterialien in OLEDs.

US 2010/171417 offenbart lochtransportierende Materialien für OLEDs. Diese bestehen aus Verbindungen, welche zwei Dihydroacridinteile aufweisen, die über eine Phenylgruppe verbunden sind.

Es besteht jedoch unverändert Bedarf an alternativen Lochtransport- und Lochinjektionsmaterialien zur Verwendung in OLEDs. Insbesondere besteht Bedarf an Materialien, mit denen die oben genannten, hoch erwünschten Verbesserungen der Leistungsdaten und Eigenschaften der OLEDs erreicht werden können.

Ebenfalls besteht Bedarf an alternativen Matrixmaterialien zur Verwendung in OLEDs sowie in anderen elektronischen Vorrichtungen. Insbesondere besteht Bedarf an Matrixmaterialien für phosphoreszierende Dotanden sowie an Matrixmaterialien für Mixed-Matrix-Systeme, welche bevorzugt zu guter Effizienz, hoher Lebensdauer und geringer Betriebsspannung der elektronischen Vorrichtungen führen.

Der vorliegenden Erfindung liegt somit die technische Aufgabe zu Grunde, Verbindungen bereitzustellen, welche sich zur Verwendung in elektronischen Vorrichtungen wie beispielsweise OLEDs eignen, und welche insbesondere als Lochtransportmaterialien und/oder als Matrixmaterialien eingesetzt werden können.

Im Rahmen der vorliegenden Erfindung wurde nun gefunden, dass sich Verbindungen der unten angegebenen Formel (I) ausgezeichnet für die oben genannten Verwendungen eignen.

Gegenstand der Erfindung ist somit eine Verbindung einer Formel (I) 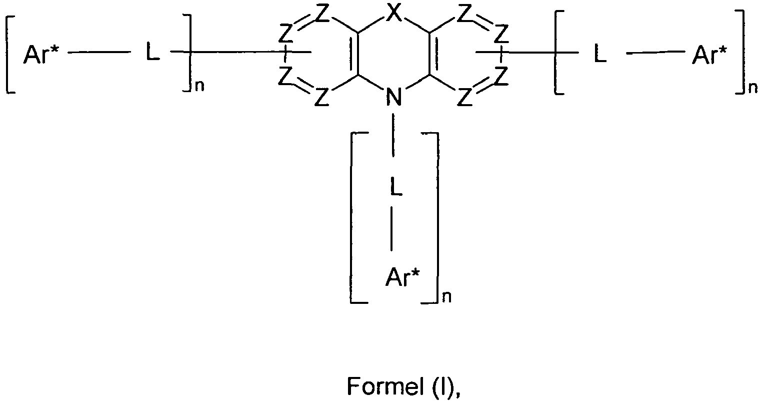 wobei für die auftretenden Symbole und Indices gilt:
- Ar*: ist bei jedem Auftreten gleich oder verschieden ein aromatisches Ringsystem mit 6 bis 24 aromatischen Ringatomen oder eine elektronenreiche Heteroarylgruppe mit 5 bis 18 aromatischen Ringatomen, welche jeweils mit einem oder mehreren Resten R² substituiert sein können;
- L: ist bei jedem Auftreten gleich oder verschieden eine Einfachbindung, C=O, C=NR², Si(R²)₂, PR², P(=O)(R²), O, S, SO, SO₂, eine Alkylengruppe mit 1 bis 20 C-Atomen oder eine Alkenylen- oder Alkinylengruppe mit 2 bis 20 C-Atomen, wobei in den genannten Gruppen eine oder mehrere CH₂-Gruppen durch C=O, C=NR², C=O-O, C=O-NR², Si(R²)₂, NR², P(=O)(R²), O, S, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 24 aromatischen Ringatomen, welches durch einen oder mehrere Reste R² substituiert sein kann, oder eine beliebige Kombination aus 1, 2, 3, 4 oder 5 gleichen oder verschiedenen Gruppen ausgewählt aus den oben genannten Gruppen;
- X: ist eine divalente Gruppe ausgewählt aus C(R¹)₂, Si(R¹)₂, NR¹, PR¹, O und S;
- Z: ist bei jedem Auftreten gleich oder verschieden CR² oder N, sofern Z keinen Substituenten trägt, und ist gleich C, sofern Z einen Substituenten trägt;
- R¹: ist bei jedem Auftreten gleich oder verschieden H, D, F, Cl, Br, I, C(=O)R³, CN, Si(R³)₃, NO₂, P(=O)(R³)₂, S=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R³ substituiert sein kann, wobei zwei oder mehr nicht-aromatische Reste R¹ miteinander verknüpft sein können und einen Ring bilden können;
- R²: ist bei jedem Auftreten gleich oder verschieden H, D, F, Cl, Br, I, C(=O)R³, CN, Si(R³)₃, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei oder mehr Reste R² miteinander verknüpft sein können und einen Ring bilden können;
- R³: ist bei jedem Auftreten gleich oder verschieden H, D, F, Cl, Br, I, C(=O)R⁴, CN, Si(R⁴)₃, NO₂, P(=O)(R⁴)₂, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
- R⁴: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
- n: ist bei jedem Auftreten gleich oder verschieden 0 oder 1, wobei mindestens zwei Indices n gleich 1 sein müssen; für n=0 ist an der entsprechenden Position eine Gruppe R² gebunden;
wobei die Verbindung keine Carbazolgruppe umfasst; und
wobei mindestens eine Gruppe Ar* in der Verbindung enthalten sein muss, welche eine elektronenreiche Heteroarylgruppe mit 5 bis 18 aromatischen Ringatomen oder ein aromatisches Ringsystem mit 12 bis 24 aromatischen Ringatomen darstellt.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

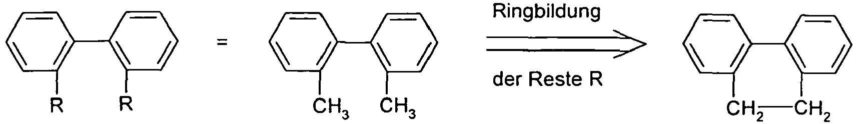

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

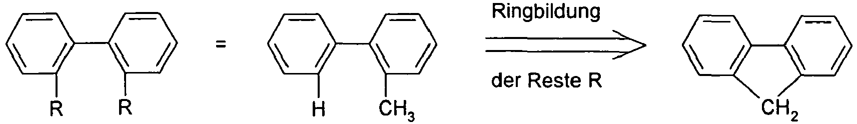

Zur Klarstellung soll angemerkt werden, dass die Formulierung "nicht-aromatische Reste" in der Definition von R¹ sich auf jegliche Arten von aromatischen Gruppen, u. a. Arylgruppen und aromatische Ringsysteme, bezieht. Hierdurch wird beispielsweise ausgeschlossen, dass die Gruppe X in der Ausführungsform C(R¹)₂ oder Si(R¹)₂ ein Spirozentrum darstellt, welches an vier aromatische Ringe bindet, wie dies in einem Spirobifluorenderivat der Fall ist.

Unter dem Begriff "elektronenreiche Heteroarylgruppe" als Ausführungsform von Ar* wird gemäß der vorliegenden Erfindung eine Heteroarylgruppe, wie oben definiert, verstanden, welche mindestens einen heteroaromatischen Fünfring enthaltend genau ein Heteroatom umfasst, wobei Carbazolderivate nicht als elektronenreiche Heteroarylgruppen gemäß der vorliegenden Erfindung verstanden werden. Unter Carbazolderivaten werden im Sinne der vorliegenden Erfindung auch Carbazolderivate mit ankondensierten Gruppen, wie beispielsweise Indenocarbazole oder Indolocarbazole, sowie Carbazolderivate, in denen ein oder mehrere Kohlenstoffatome in den aromatischen Sechsringen durch Stickstoff ersetzt sind, verstanden.

Bevorzugte elektronenreiche Heteroarylgruppen als Gruppen Ar* gemäß der vorliegenden Erfindung sind Verbindungen der folgenden Formel (H) 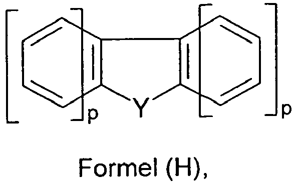 wobei
- Y: ausgewählt ist aus NR², PR², O und S; und
- p: bei jedem Auftreten gleich 0 oder 1 ist, wobei für p=0 an den betreffenden Positionen Reste R² gebunden sind, und wobei für Y=NR² nicht beide Indices p gleich 1 sein dürfen;
die Gruppe an allen freien Positionen mit Resten R² substituiert ist, und
die Gruppe an einer beliebigen Position mit der Gruppe L verbunden sein kann, wobei die Bindung auch an die Stelle der Bindung NR² oder PR² treten kann.

Besonders bevorzugt sind Gruppen der Formeln (H-1) bis (H-5) 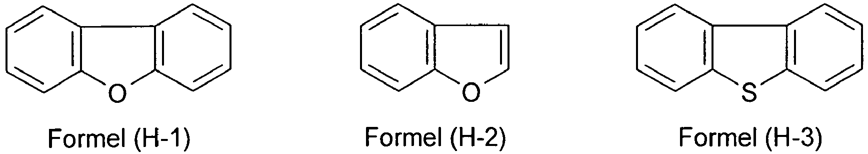 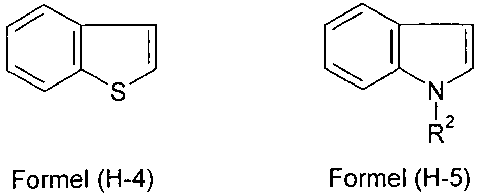 wobei die Gruppen an allen freien Positionen mit Resten R² substituiert sein können, und
die Gruppen an einer beliebigen Position mit der Gruppe L verbunden sein können, wobei die Bindung auch an die Stelle der Bindung NR² treten kann.

Bevorzugt sind die Gruppen der Formel (H-1) und (H-3) in den Positionen 1, 2, 3 oder 4 des Dibenzothiophen- oder des Dibenzofuran-Grundkörpers mit der Gruppe L verbunden.

Weitere erfindungsgemäße Ausführungsformen der Gruppe Ar* sind neben den oben definierten elektronenreichen Heteroarylgruppen aromatische Ringsysteme enthaltend 6 bis 24 aromatische Ringatome, welche mit einem oder mehreren Resten R² substituiert sein können. Bevorzugt sind aromatische Ringsysteme, welche keine kondensierten Arylgruppen mit mehr als 14 aromatischen Ringatomen enthalten und besonders bevorzugt solche, welche keine Arylgruppen mit mehr als 10 aromatischen Ringatomen enthalten. Ganz besonders bevorzugt sind aromatische Ringsysteme, welche ausschließlich Arylgruppen mit 6 aromatischen Ringatomen (=Phenylgruppen) umfassen. Nochmals stärker bevorzugt stellt Ar* eine Phenylgruppe, eine Biphenylgruppe oder eine Terphenylgruppe dar.

Bevorzugte aromatische Ringsysteme als Gruppen Ar* werden durch die folgenden Formeln wiedergegeben: 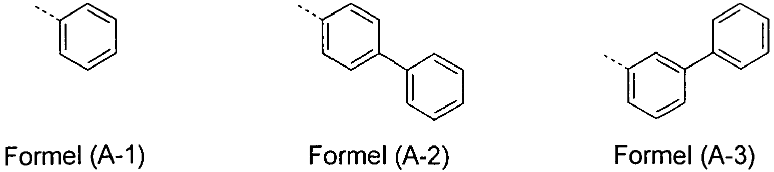 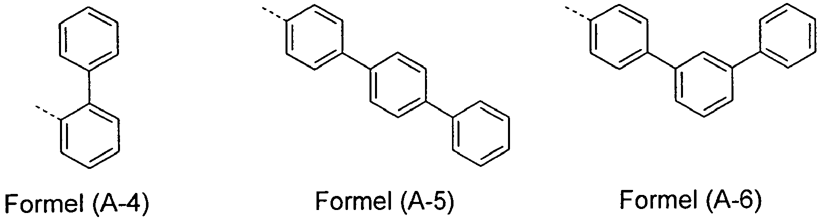 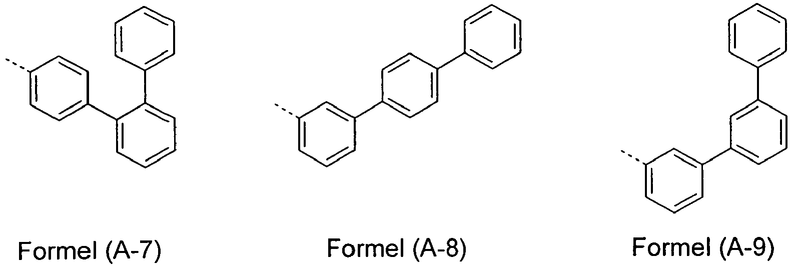 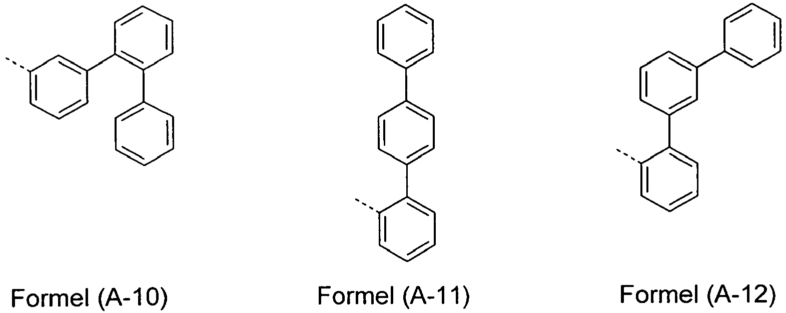 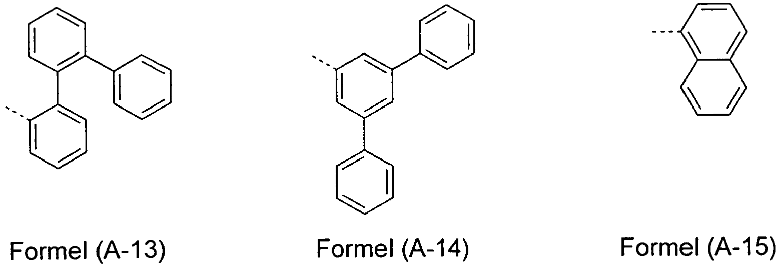 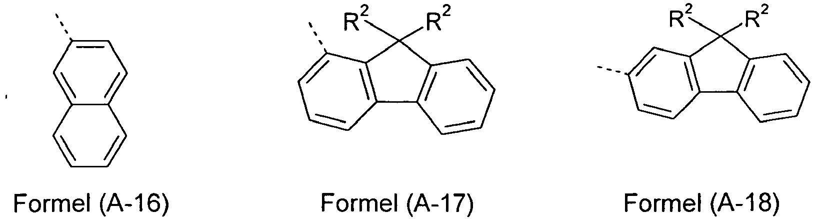 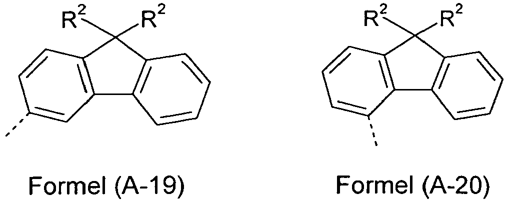 wobei die Strukturen mit einem oder mehreren Resten R² substituiert sein können, und R² definiert ist wie oben angegeben.

Bevorzugt sind die Gruppen der Formel (A-1) bis (A-14) in mindestens einer ortho-Position einer Phenylgruppe mit einem Rest R² ausgewählt aus F und einer Alkylgruppe mit 1 bis 10 C-Atomen substituiert, besonders bevorzugt mit einem Rest R² ausgewählt aus F und einer Alkylgruppe mit 1 bis 5 C-Atomen, ganz besonders bevorzugt mit einem Rest ausgewählt aus F und Methyl.

In einer bevorzugten Ausführungsform der Erfindung ist die Gruppe L bei jedem Auftreten gleich oder verschieden eine Einfachbindung, Si(R²)₂, O, S, eine Alkylengruppe mit 1 bis 10 C-Atomen oder eine Alkenylen- oder Alkinylengruppe mit 2 bis 10 C-Atomen, wobei bei den genannten Gruppen eine oder mehrere CH₂-Gruppen durch Si(R²)₂, O oder S ersetzt sein können und wobei ein oder mehrere H-Atome in den genannten Gruppen durch D, F oder CN ersetzt sein können, oder ein aromatisches Ringsystem mit 6 bis 24 aromatischen Ringatomen, welches durch einen oder mehrere Reste R² substituiert sein kann.

Besonders bevorzugt ist L bei jedem Auftreten gleich oder verschieden eine Einfachbindung oder ein aromatisches Ringsystem mit 6 bis 12 aromatischen Ringatomen, welches durch einen oder mehrere Reste R² substituiert sein kann.

Ganz besonders bevorzugt ist L eine Einfachbindung.

Gemäß einer bevorzugten Ausführungsform ist genau eine Gruppe L-Ar* an das Stickstoffatom des Grundgerüsts gemäß Formel (I) gebunden, und genau eine weitere Gruppe L-Ar* ist an den aromatischen Sechsring des Grundgerüsts gemäß Formel (I) gebunden. Dies entspricht einer Ausführungsform, in der genau zwei der drei in Formel (I) vorhandenen Indices n gleich 1 sind und genau einer der Indices n gleich Null ist. Gemäß einer weiteren bevorzugten Ausführungsform sind alle Indices n gleich 1, so dass sowohl an das Stickstoffatom als auch an die beiden aromatischen Sechsringe des Grundgerüsts gemäß Formel (I) je eine Gruppe L-Ar* gebunden ist.

Weiterhin ist es bevorzugt, dass die Anbindungsposition der Gruppe L-Ar* an den aromatischen Sechsring des Grundgerüsts gemäß Formel (I) in der Position para oder meta zum Stickstoffatom vorliegt, besonders bevorzugt in der Position para zum Stickstoffatom.

Es ist erfindungsgemäß bevorzugt, dass in der Gruppe L-Ar*, welche an das Stickstoffatom gebunden ist, L eine Einfachbindung darstellt und Ar* ein aromatisches Ringsystem mit 6 bis 24 aromatischen Ringatomen darstellt, welches mit einem oder mehreren Resten R² substituiert sein kann. Besonders bevorzugt ist Ar* in diesem Fall ein aromatisches Ringsystem mit 6 bis 18 aromatischen Ringatomen, ganz besonders bevorzugt ein aromatisches Ringsystem mit 12 bis 18 aromatischen Ringatomen, welches mit einem oder mehreren Resten R² substituiert sein kann.

Weiterhin ist es erfindungsgemäß bevorzugt, dass X ausgewählt ist aus C(R¹)₂, O und S. Besonders bevorzugt ist X gleich C(R¹)₂.

Weiterhin bevorzugt ist maximal eine Gruppe Z pro aromatischem Sechsring des Grundgerüsts gemäß Formel (I) gleich N. Besonders bevorzugt ist keine Gruppe Z gleich N, so dass alle Gruppen Z gleich C sind, wenn ein Substituent gebunden ist, und gleich CR² sind, wenn kein Substituent gebunden ist.

Gemäß einer bevorzugten Ausführungsform ist R¹ bei jedem Auftreten gleich oder verschieden H, D, F, eine geradkettige Alkyl-, Alkoxy- oder Thioalkylgruppe mit 1 bis 10 C-Atomen oder eine verzweigte oder cyclische Alkyl-, Alkoxy- oder Thioalkylgruppe mit 3 bis 10 C-Atomen oder eine Alkenyl- oder Alkinylgruppe mit 2 bis 10 C-Atomen, wobei die oben genannten Gruppen jeweils mit einem oder mehreren Resten R³ substituiert sein können und wobei eine oder mehrere CH₂-Gruppen in den oben genannten Gruppen durch Si(R³)₂, O oder S ersetzt sein können und wobei ein oder mehrere H-Atome in den oben genannten Gruppen durch D, F oder CN ersetzt sein können, oder ein aromatisches Ringsystem mit 6 bis 18 aromatischen Ringatomen, das jeweils durch einen oder mehrere Reste R³ substituiert sein kann, wobei zwei oder mehr Alkyl-, Alkoxy- oder Thioalkylgruppen R¹ miteinander verknüpft sein können und einen Ring bilden können.

Besonders bevorzugt ist R¹ bei jedem Auftreten gleich oder verschieden H, D, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können, oder ein aromatisches Ringsystem mit 6 bis 12 aromatischen Ringatomen, das jeweils durch einen oder mehrere Reste R³ substituiert sein kann, wobei zwei oder mehr Alkyl- oder Alkoxygruppen R¹ miteinander verknüpft sein können und einen Ring bilden können.

Weiterhin ist es erfindungsgemäß bevorzugt, dass Reste R¹ an einer Gruppe X = C(R¹)₂, miteinander einen Cycloalkylring bilden. Besonders bevorzugt sind ein Cyclohexyl- und ein Cyclopentylring, welche jeweils mit einer oder mehreren Resten R³ substituiert sein können.

Bevorzugt ist weiterhin mindestens eine Gruppe R¹ in einer Gruppe X = C(R¹)₂ in der erfindungsgemäßen Verbindung keine aromatische Gruppe, d. h. kein aromatisches Ringsystem und keine Arylgruppe. Besonders bevorzugt stellen die Gruppen R¹ in einer Gruppe X = C(R¹)₂ in der erfindungsgemäßen Verbindung kein aromatisches Ringsystem und keine Arylgruppe dar.

Gemäß einer bevorzugten Ausführungsform ist R² bei jedem Auftreten gleich oder verschieden H, D, F, CN, Si(R³)₃, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch Si(R³)₂, O und S ersetzt sein können, oder ein aromatisches oder heteroaromatisches Ringsystem mit 5 bis 18 aromatischen Ringatomen, das jeweils mit einem oder mehreren Resten R³ substituiert sein kann, wobei zwei oder mehr Reste R² miteinander verknüpft sein können und einen Ring bilden können.

Gemäß einer weiteren bevorzugten Ausführungsform ist R³ bei jedem Auftreten gleich oder verschieden H, D, F, CN, Si(R⁴)₃ oder eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R⁴ substituiert sein können und wobei in den oben genannten Gruppen eine oder mehrere CH₂-Gruppen durch Si(R⁴)₂, O und S ersetzt sein können, oder ein aromatisches oder heteroaromatisches Ringsystem mit 5 bis 18 aromatischen Ringatomen, das jeweils mit einem oder mehreren Resten R⁴ substituiert sein kann, wobei zwei oder mehr Reste R³ miteinander verknüpft sein können und einen Ring bilden können.

Gemäß einer weiteren bevorzugten Ausführungsform enthält die erfindungsgemäße Verbindung keine Heteroarylgruppe ausgewählt aus Triazin, Pyrimidin, Pyrazin, Pyridazin, Pyridin, Imidazol und Benzimidazol sowie keine Ketogruppe, keine Phosphoroxidgruppe und keine Schwefeloxidgruppe. Besonders bevorzugt enthält die erfindungsgemäße Verbindung keine elektronenarme Heteroarylgruppe sowie keine Ketogruppe, keine Phosphoroxidgruppe und keine Schwefeloxidgruppe. Unter einer elektronenarmen Heteroarylgruppe werden im Rahmen der vorliegenden Erfindung insbesondere heteroaromatische Sechsringe mit einem oder mehreren Stickstoffatomen und heteroaromatische Fünfringe mit zwei oder mehr Heteroatomen, insbesondere Heteroatomen ausgewählt aus N, O und S, verstanden.

Gemäß einer weiteren bevorzugten Ausführungsform umfasst die erfindungsgemäße Verbindung zusätzlich zur Aminogruppe der Grundstruktur keine weitere Arylaminogruppe.

Bevorzugte Ausführungsformen der erfindungsgemäßen Verbindung der Formel (I) werden durch die folgenden Formeln (I-A), (I-B) und (I-C) wiedergegeben: 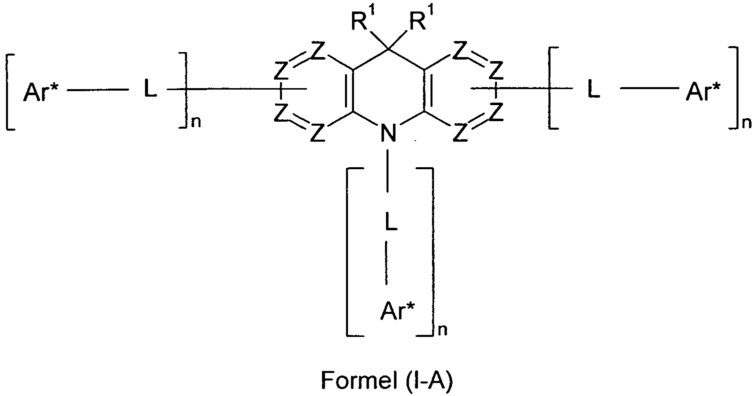 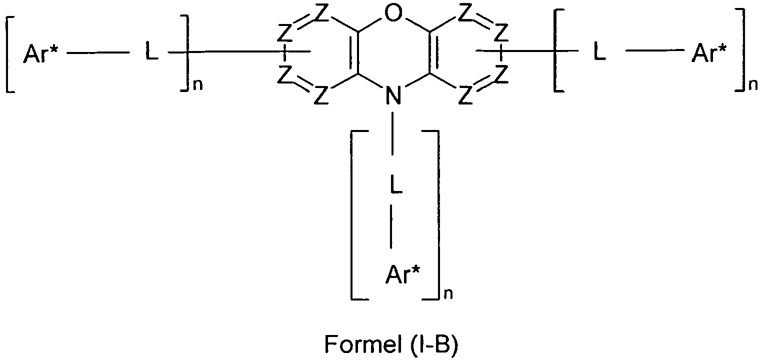 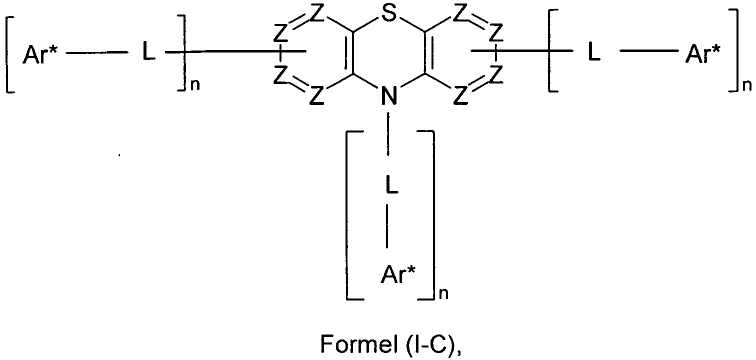 wobei die auftretenden Symbole und Indices wie oben definiert sind. Bevorzugt ist die Kombination der oben aufgeführten bevorzugten Ausführungsformen der Gruppen R¹, Ar*, L und Z und des Index n mit den Strukturen der Formeln (I-A) bis (I-C).

Bevorzugte Ausführungsformen der Formel (I-A) sind die Formeln (I-A-1) und (I-A-2) 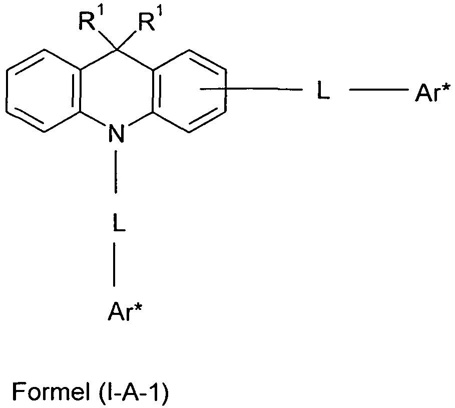 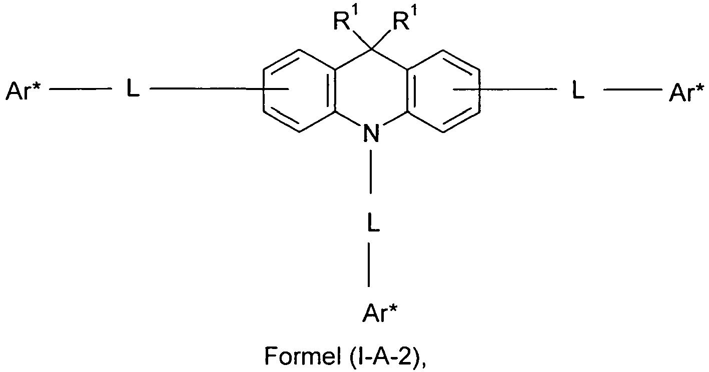 wobei die auftretenden Symbole definiert sind wie oben angegeben und die freien Positionen an den aromatischen Sechsringen mit Resten R² substituiert sein können.

Bevorzugt ist die Kombination der oben aufgeführten bevorzugten Ausführungsformen der Gruppen R¹, Ar* und L mit den Strukturen der Formeln (I-A-1) und (I-A-2). Weiterhin bevorzugt ist es, dass die Gruppen L-Ar* in den Positionen meta oder para zum Stickstoffatom gebunden sind, besonders bevorzugt in der Position para zum Stickstoffatom.

Bevorzugte Ausführungsformen der Formel (I-B) sind die Formeln (I-B-1) und (I-B-2) 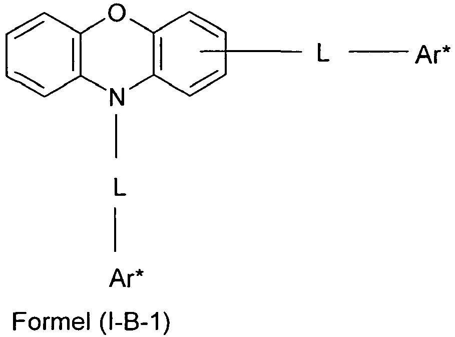 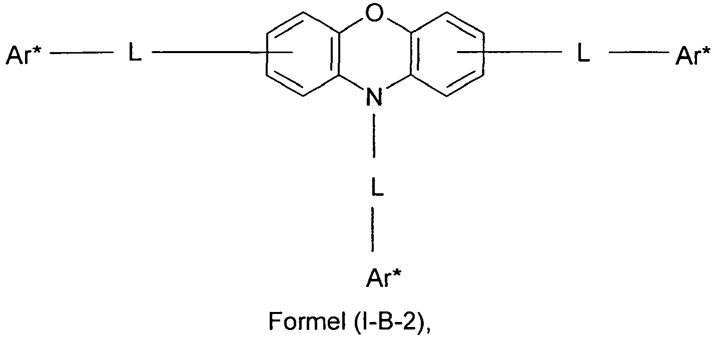 wobei die auftretenden Symbole definiert sind wie oben angegeben und die freien Positionen an den aromatischen Sechsringen mit Resten R² substituiert sein können.

Bevorzugt ist die Kombination der oben aufgeführten bevorzugten Ausführungsformen der Gruppen Ar* und L mit den Strukturen der Formeln (I-B-1) und (I-B-2). Weiterhin bevorzugt ist es, dass die Gruppen L-Ar* in den Positionen meta oder para zum Stickstoffatom gebunden sind, besonders bevorzugt in der Position para zum Stickstoffatom.

Bevorzugte Ausführungsformen der Formel (I-C) sind die Formeln (I-C-1) und (I-C-2) 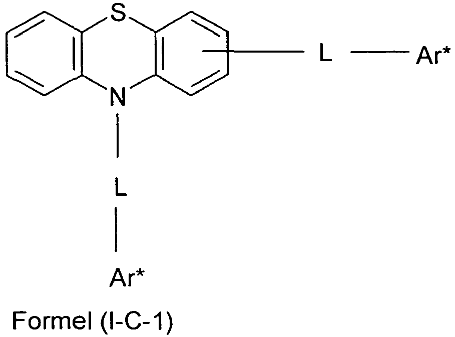 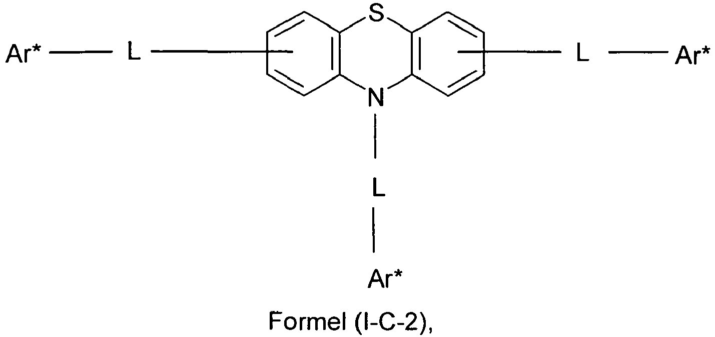 wobei die auftretenden Symbole definiert sind wie oben angegeben und die freien Positionen an den aromatischen Sechsringen mit Resten R² substituiert sein können.

Bevorzugt ist die Kombination der oben aufgeführten bevorzugten Ausführungsformen der Gruppen Ar* und L mit den Strukturen der Formeln (I-C-1) und (I-C-2). Weiterhin bevorzugt ist es, dass die Gruppen L-Ar* in den Positionen meta oder para zum Stickstoffatom gebunden sind, besonders bevorzugt in der Position para zum Stickstoffatom.

Weiterhin sind folgende Kombinationen von Gruppen Ar* der Formeln (H-1), (H-3), (A-1) und (A-2) mit den bevorzugten Strukturen der Formeln (I-A-1), (I-A-2), (I-B-1), (I-B-2), (I-C-1) und (I-C-2) bevorzugt (L ist in allen in der Tabelle aufgeführten Fällen eine Einfachbindung):

Die unterschiedlichen auftretenden Gruppen L-Ar* werden dabei wie folgt gekennzeichnet (gezeigt am Beispiel der Formel (I-A-2):

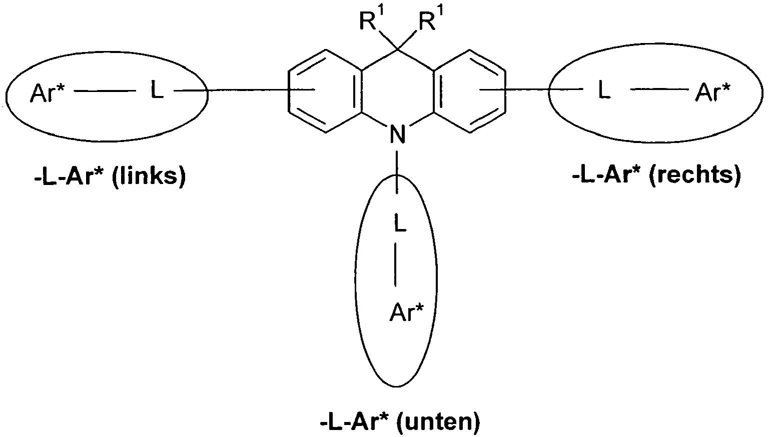

| | **Grundstruktur** | **-L-Ar* (links)** | **-L-Ar* (rechts)** | **-L-Ar* (unten)** |
|---|---|---|---|---|
| (I-A-1-1) | (I-A-1) | -- | (H-1) | (H-1) |
| (I-A-1-2) | s. o. | -- | (H-1) | (H-3) |
| (I-A-1-3) | s. o. | -- | (H-1) | (A-1) |
| (I-A-1-4) | s. o. | -- | (H-1) | (A-2) |
| (I-A-1-5) | s. o. | -- | (H-3) | (H-1) |
| (I-A-1-6) | s. o. | -- | (H-3) | (H-3) |
| (I-A-1-7) | s. o. | -- | (H-3) | (A-1) |
| (I-A-1-8) | s. o. | -- | (H-3) | (A-2) |
| (I-A-1-9) | s. o. | -- | (A-1) | (H-1) |
| (I-A-1-10) | s. o. | -- | (A-1) | (H-3) |
| (I-A-1-11) | s. o. | -- | (A-1) | (A-1) |
| (I-A-1-12) | s. o. | -- | (A-1) | (A-2) |
| (I-A-1-13) | s. o. | -- | (A-2) | (H-1) |
| (I-A-1-14) | s. o. | -- | (A-2) | (H-3) |
| (I-A-1-15) | s. o. | -- | (A-2) | (A-1) |
| (I-A-1-16) | s. o. | -- | (A-2) | (A-2) |
| (I-A-2-1) | (I-A-2) | (H-1) | (H-1) | (H-1) |
| (I-A-2-2) | s. o. | (H-1) | (H-3) | (H-1) |
| (I-A-2-3) | s. o. | (H-1) | (A-1) | (H-1) |
| (I-A-2-4) | s. o. | (H-1) | (A-2) | (H-1) |
| (I-A-2-5) | s. o. | (H-3) | (H-3) | (H-1) |
| (I-A-2-6) | s. o. | (H-3) | (A-1) | (H-1) |
| (I-A-2-7) | s. o. | (H-3) | (A-2) | (H-1) |
| (I-A-2-8) | s. o. | (A-1) | (A-1) | (H-1) |
| (I-A-2-9) | s. o. | (A-1) | (A-2) | (H-1) |
| (I-A-2-10 | s. o. | (A-2) | (A-2) | (H-1) |
| (I-A-2-11) | s. o. | (H-1) | (H-1) | (H-3) |
| (I-A-2-12) | s. o. | (H-1) | (H-3) | (H-3) |
| (I-A-2-13) | s. o. | (H-1) | (A-1) | (H-3) |
| (I-A-2-14) | s. o. | (H-1) | (A-2) | (H-3) |
| (I-A-2-15) | s. o. | (H-3) | (H-3) | (H-3) |
| (I-A-2-16) | s. o. | (H-3) | (A-1) | (H-3) |
| (I-A-2-17) | s. o. | (H-3) | (A-2) | (H-3) |
| (I-A-2-18) | s. o. | (A-1) | (A-1) | (H-3) |
| (I-A-2-19) | s. o. | (A-1) | (A-2) | (H-3) |
| (I-A-2-20) | s. o. | (A-2) | (A-2) | (H-3) |
| (I-A-2-21) | s. o. | (H-1) | (H-1) | (A-1) |
| (I-A-2-22) | s. o. | (H-1) | (H-3) | (A-1) |
| (I-A-2-23) | s. o. | (H-1) | (A-1) | (A-1) |
| (I-A-2-24) | s. o. | (H-1) | (A-2) | (A-1) |
| (I-A-2-25) | s. o. | (H-3) | (H-3) | (A-1) |
| (I-A-2-26) | s. o. | (H-3) | (A-1) | (A-1) |
| (I-A-2-27) | s. o. | (H-3) | (A-2) | (A-1) |
| (I-A-2-28) | s. o. | (A-1) | (A-1) | (A-1) |
| (I-A-2-29) | s. o. | (A-1) | (A-2) | (A-1) |
| (I-A-2-30) | s. o. | (A-2) | (A-2) | (A-1) |
| (I-A-2-31) | s. o. | (H-1) | (H-1) | (A-2) |
| (I-A-2-32) | s. o. | (H-1) | (H-3) | (A-2) |
| (I-A-2-33) | s. o. | (H-1) | (A-1) | (A-2) |
| (I-A-2-34) | s. o. | (H-1) | (A-2) | (A-2) |
| (I-A-2-35) | s. o. | (H-3) | (H-3) | (A-2) |
| (I-A-2-36) | s. o. | (H-3) | (A-1) | (A-2) |
| (I-A-2-37) | s. o. | (H-3) | (A-2) | (A-2) |
| (I-A-2-38) | s. o. | (A-1) | (A-1) | (A-2) |
| (I-A-2-39) | s. o. | (A-1) | (A-2) | (A-2) |
| (I-A-2-40) | s. o. | (A-2) | (A-2) | (A-2) |
| (I-B-1-1) | (I-B-1) | -- | (H-1) | (H-1) |
| (I-B-1-2) | s. o. | -- | (H-1) | (H-3) |
| (I-B-1-3) | s. o. | -- | (H-1) | (A-1) |
| (I-B-1-4) | s. o. | -- | (H-1) | (A-2) |
| (I-B-1-5) | s. o. | -- | (H-3) | (H-1) |
| (I-B-1-6) | s. o. | -- | (H-3) | (H-3) |
| (I-B-1-7) | s. o. | -- | (H-3) | (A-1) |
| (I-B-1-8) | s. o. | -- | (H-3) | (A-2) |
| (I-B-1-9) | s. o. | -- | (A-1) | (H-1) |
| (I-B-1-10) | s. o. | -- | (A-1) | (H-3) |
| (I-B-1-11) | s. o. | -- | (A-1) | (A-1) |
| (I-B-1-12) | s. o. | -- | (A-1) | (A-2) |
| (I-B-1-13) | s. o. | -- | (A-2) | (H-1) |
| (I-B-1-14) | s. o. | -- | (A-2) | (H-3) |
| (I-B-1-15) | s. o. | -- | (A-2) | (A-1) |
| (I-B-1-16) | s. o. | -- | (A-2) | (A-2) |
| (I-B-2-1) | (I-B-2) | (H-1) | (H-1) | (H-1) |
| (I-B-2-2) | s. o. | (H-1) | (H-3) | (H-1) |
| (I-B-2-3) | s. o. | (H-1) | (A-1) | (H-1) |
| (I-B-2-4) | s. o. | (H-1) | (A-2) | (H-1) |
| (I-B-2-5) | s. o. | (H-3) | (H-3) | (H-1) |
| (I-B-2-6) | s. o. | (H-3) | (A-1) | (H-1) |
| (I-B-2-7) | s. o. | (H-3) | (A-2) | (H-1) |
| (I-B-2-8) | s. o. | (A-1) | (A-1) | (H-1) |
| (I-B-2-9) | s. o. | (A-1) | (A-2) | (H-1) |
| (I-B-2-10 | s. o. | (A-2) | (A-2) | (H-1) |
| (I-B-2-11) | s. o. | (H-1) | (H-1) | (H-3) |
| (I-B-2-12) | s. o. | (H-1) | (H-3) | (H-3) |
| (I-B-2-13) | s. o. | (H-1) | (A-1) | (H-3) |
| (I-B-2-14) | s. o. | (H-1) | (A-2) | (H-3) |
| (I-B-2-15) | s. o. | (H-3) | (H-3) | (H-3) |
| (I-B-2-16) | s. o. | (H-3) | (A-1) | (H-3) |
| (I-B-2-17) | s. o. | (H-3) | (A-2) | (H-3) |
| (I-B-2-18) | s. o. | (A-1) | (A-1) | (H-3) |
| (I-B-2-19) | s. o. | (A-1) | (A-2) | (H-3) |
| (I-B-2-20) | s. o. | (A-2) | (A-2) | (H-3) |
| (I-B-2-21) | s. o. | (H-1) | (H-1) | (A-1) |
| (I-B-2-22) | s. o. | (H-1) | (H-3) | (A-1) |
| (I-B-2-23) | s. o. | (H-1) | (A-1) | (A-1) |
| (I-B-2-24) | s. o. | (H-1) | (A-2) | (A-1) |
| (I-B-2-25) | s. o. | (H-3) | (H-3) | (A-1) |
| (I-B-2-26) | s. o. | (H-3) | (A-1) | (A-1) |
| (I-B-2-27) | s. o. | (H-3) | (A-2) | (A-1) |
| (I-B-2-28) | s. o. | (A-1) | (A-1) | (A-1) |
| (I-B-2-29) | s. o. | (A-1) | (A-2) | (A-1) |
| (I-B-2-30) | s. o. | (A-2) | (A-2) | (A-1) |
| (I-B-2-31) | s. o. | (H-1) | (H-1) | (A-2) |
| (I-B-2-32) | s. o. | (H-1) | (H-3) | (A-2) |
| (I-B-2-33) | s. o. | (H-1) | (A-1) | (A-2) |
| (I-B-2-34) | s. o. | (H-1) | (A-2) | (A-2) |
| (I-B-2-35) | s. o. | (H-3) | (H-3) | (A-2) |
| (I-B-2-36) | s. o. | (H-3) | (A-1) | (A-2) |
| (I-B-2-37) | s. o. | (H-3) | (A-2) | (A-2) |
| (I-B-2-38) | s. o. | (A-1) | (A-1) | (A-2) |
| (I-B-2-39) | s. o. | (A-1) | (A-2) | (A-2) |
| (I-B-2-40) | s. o. | (A-2) | (A-2) | (A-2) |
| (I-C-1-1) | (I-C-1) | -- | (H-1) | (H-1) |
| (I-C-1-2) | s. o. | -- | (H-1) | (H-3) |
| (I-C-1-3) | s. o. | -- | (H-1) | (A-1) |
| (I-C-1-4) | s. o. | -- | (H-1) | (A-2) |
| (I-C-1-5) | s. o. | -- | (H-3) | (H-1) |
| (I-C-1-6) | s. o. | -- | (H-3) | (H-3) |
| (I-C-1-7) | s. o. | -- | (H-3) | (A-1) |
| (I-C-1-8) | s. o. | -- | (H-3) | (A-2) |
| (I-C-1-9) | s. o. | -- | (A-1) | (H-1) |
| (I-C-1-10) | s. o. | -- | (A-1) | (H-3) |
| (I-C-1-11) | s. o. | -- | (A-1) | (A-1) |
| (I-C-1-12) | s. o. | -- | (A-1) | (A-2) |
| (I-C-1-13) | s. o. | -- | (A-2) | (H-1) |
| (I-C-1-14) | s. o. | -- | (A-2) | (H-3) |
| (I-C-1-15) | s. o. | -- | (A-2) | (A-1) |
| (I-C-1-16) | s. o. | -- | (A-2) | (A-2) |
| (I-C-2-1) | (I-C-2) | (H-1) | (H-1) | (H-1) |
| (I-C-2-2) | s. o. | (H-1) | (H-3) | (H-1) |
| (I-C-2-3) | s. o. | (H-1) | (A-1) | (H-1) |
| (I-C-2-4) | s. o. | (H-1) | (A-2) | (H-1) |
| (I-C-2-5) | s. o. | (H-3) | (H-3) | (H-1) |
| (I-C-2-6) | s. o. | (H-3) | (A-1) | (H-1) |
| (I-C-2-7) | s. o. | (H-3) | (A-2) | (H-1) |
| (I-C-2-8) | s. o. | (A-1) | (A-1) | (H-1) |
| (I-C-2-9) | s. o. | (A-1) | (A-2) | (H-1) |
| (I-C-2-10 | s. o. | (A-2) | (A-2) | (H-1) |
| (I-C-2-11) | s. o. | (H-1) | (H-1) | (H-3) |
| (I-C-2-12) | s. o. | (H-1) | (H-3) | (H-3) |
| (I-C-2-13) | s. o. | (H-1) | (A-1) | (H-3) |
| (I-C-2-14) | s. o. | (H-1) | (A-2) | (H-3) |
| (I-C-2-15) | s. o. | (H-3) | (H-3) | (H-3) |
| (I-C-2-16) | s. o. | (H-3) | (A-1) | (H-3) |
| (I-C-2-17) | s. o. | (H-3) | (A-2) | (H-3) |
| (I-C-2-18) | s. o. | (A-1) | (A-1) | (H-3) |
| (I-C-2-19) | s. o. | (A-1) | (A-2) | (H-3) |
| (I-C-2-20) | s. o. | (A-2) | (A-2) | (H-3) |
| (I-C-2-21) | s. o. | (H-1) | (H-1) | (A-1) |
| (I-C-2-22) | s. o. | (H-1) | (H-3) | (A-1) |
| (I-C-2-23) | s. o. | (H-1) | (A-1) | (A-1) |
| (I-C-2-24) | s. o. | (H-1) | (A-2) | (A-1) |
| (I-C-2-25) | s. o. | (H-3) | (H-3) | (A-1) |
| (I-C-2-26) | s. o. | (H-3) | (A-1) | (A-1) |
| (I-C-2-27) | s. o. | (H-3) | (A-2) | (A-1) |
| (I-C-2-28) | s. o. | (A-1) | (A-1) | (A-1) |
| (I-C-2-29) | s. o. | (A-1) | (A-2) | (A-1) |
| (I-C-2-30) | s. o. | (A-2) | (A-2) | (A-1) |
| (I-C-2-31) | s. o. | (H-1) | (H-1) | (A-2) |
| (I-C-2-32) | s. o. | (H-1) | (H-3) | (A-2) |
| (I-C-2-33) | s. o. | (H-1) | (A-1) | (A-2) |
| (I-C-2-34) | s. o. | (H-1) | (A-2) | (A-2) |
| (I-C-2-35) | s. o. | (H-3) | (H-3) | (A-2) |
| (I-C-2-36) | s. o. | (H-3) | (A-1) | (A-2) |
| (I-C-2-37) | s. o. | (H-3) | (A-2) | (A-2) |
| (I-C-2-38) | s. o. | (A-1) | (A-1) | (A-2) |
| (I-C-2-39) | s. o. | (A-1) | (A-2) | (A-2) |
| (I-C-2-40) | s. o. | (A-2) | (A-2) | (A-2) |

Die in der Tabelle genannten Verbindungen können mit Resten R¹ und R², wie oben definiert, substituiert sein. Bevorzugt sind in diesem Zusammenhang die oben angegebenen bevorzugten Ausführungsformen der Reste R¹ und R².

Beispiele für erfindungsgemäße Verbindungen sind in der folgenden Tabelle wiedergegeben.

| | |
|---|---|
| 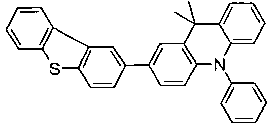 | 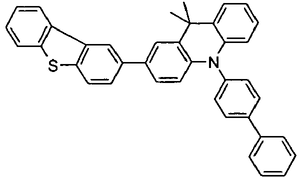 |
| 1 | 2 |
| 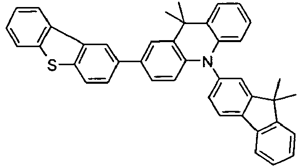 | 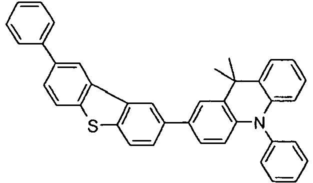 |
| 3 | 4 |
| 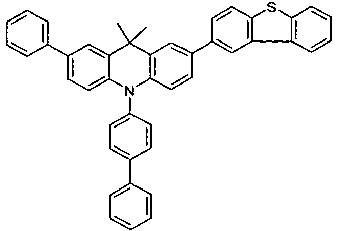 | 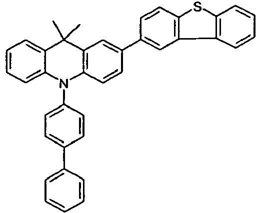 |
| 5 | 6 |
| 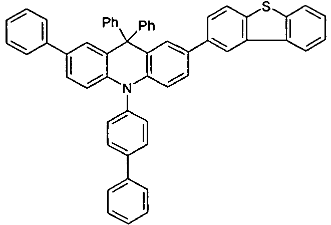 | 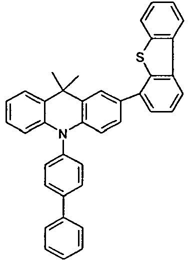 |
| 7 | 8 |
| 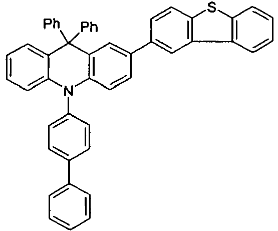 | 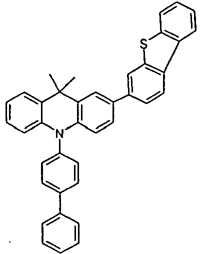 |
| 9 | 10 |
| 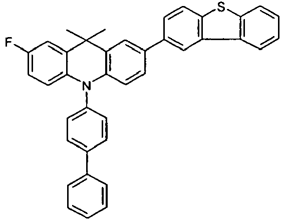 | 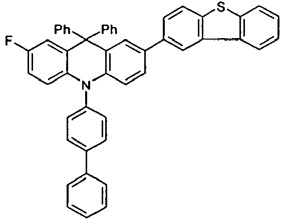 |
| 11 | 12 |
| 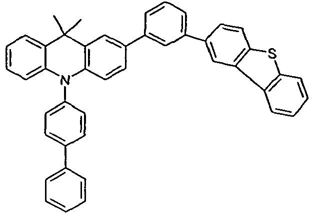 | 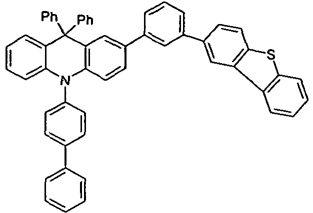 |
| 13 | 14 |
| 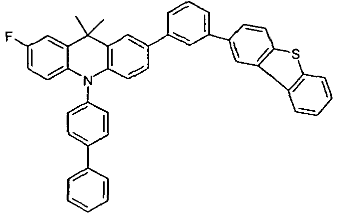 | 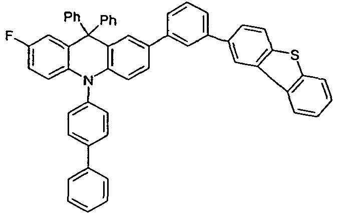 |
| 15 | 16 |
| 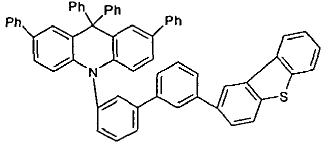 | 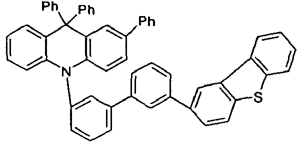 |
| 17 | 18 |
| 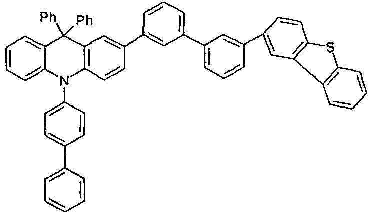 | 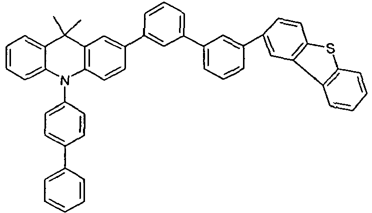 |
| 19 | 20 |
| 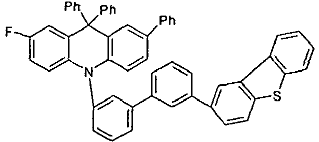 | 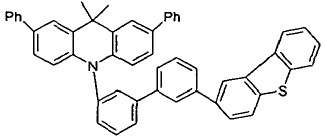 |
| 21 | 22 |
| 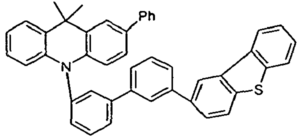 | 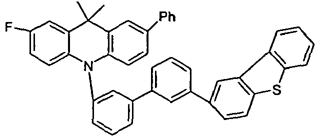 |
| 23 | 24 |
| 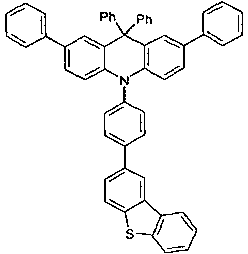 | 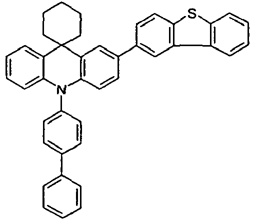 |
| 25 | 26 |
| 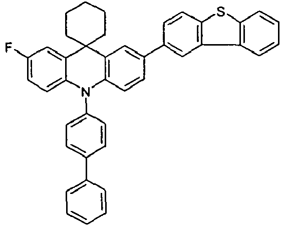 | 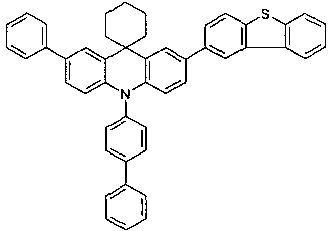 |
| 27 | 28 |
| 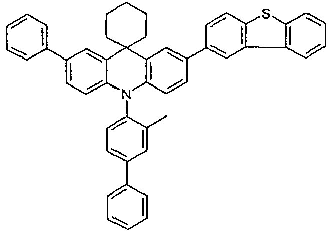 | 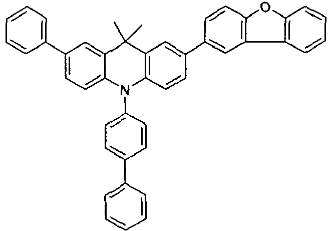 |
| 29 | 30 |
| 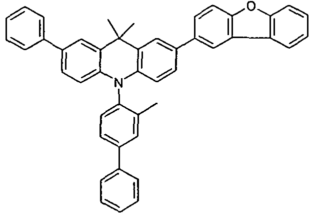 | 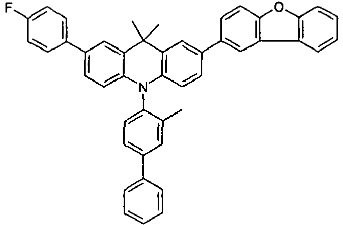 |
| 31 | 32 |
| 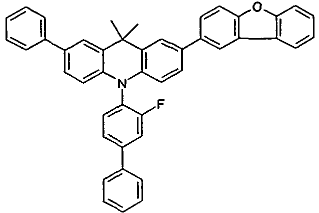 | 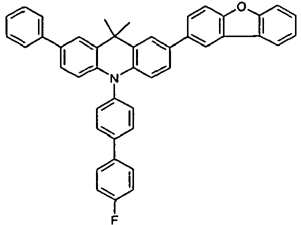 |
| 33 | 34 |
| 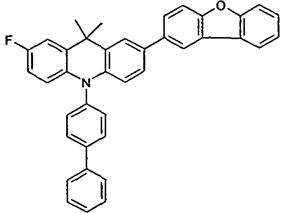 | 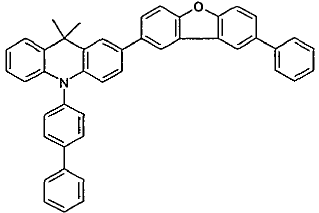 |
| 35 | 36 |
| 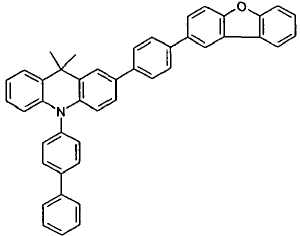 | 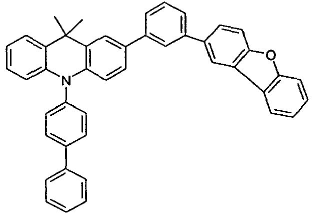 |
| 37 | 38 |
| 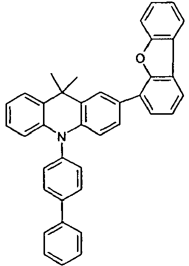 | 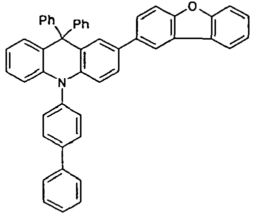 |
| 39 | 40 |
| 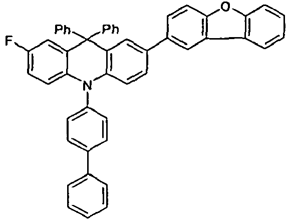 | 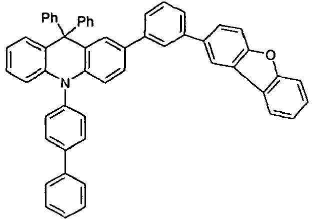 |
| 41 | 42 |
| 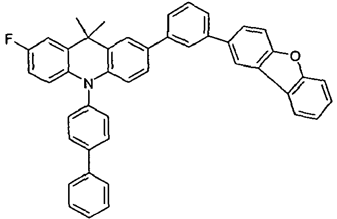 | 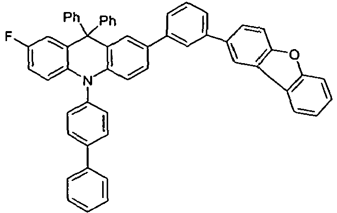 |
| 43 | 44 |
| 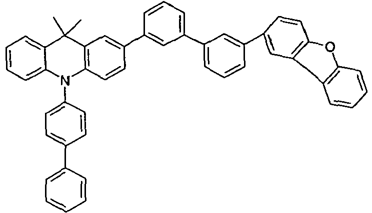 | 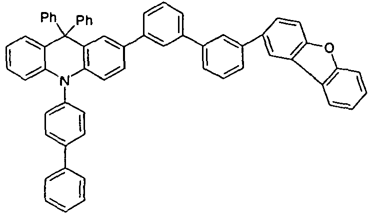 |
| 45 | 46 |
| 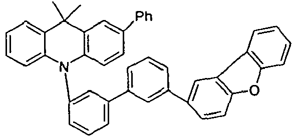 | 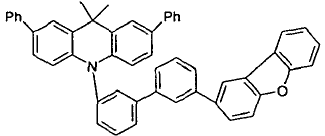 |
| 47 | 48 |
| 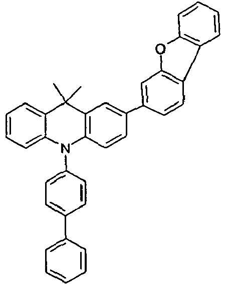 | 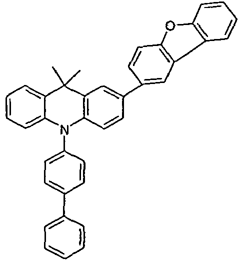 |
| 49 | 50 |
| 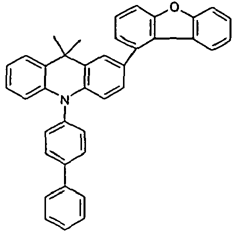 | 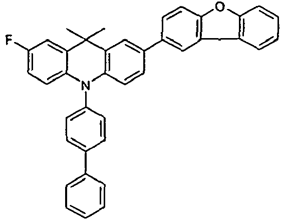 |
| 51 | 52 |
| 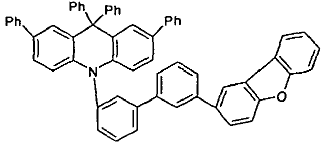 | 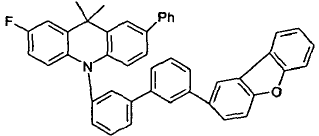 |
| 53 | 54 |
| 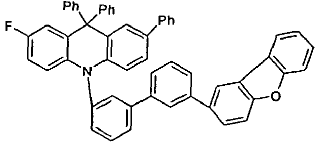 | 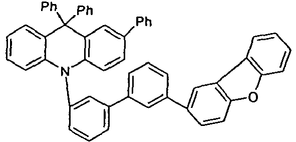 |
| 55 | 56 |
| 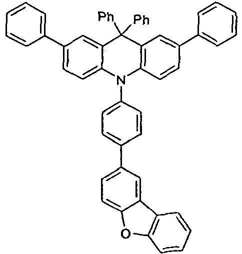 | 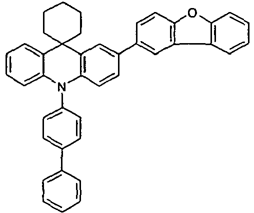 |
| 57 | 58 |
| 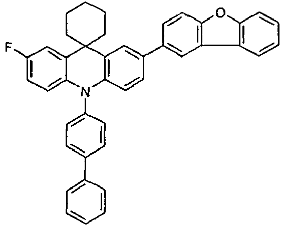 | 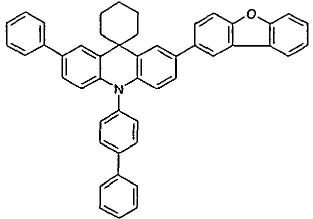 |
| 59 | 60 |
| 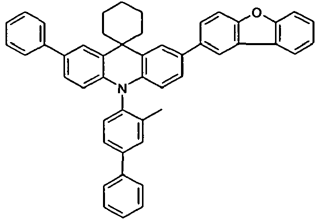 | 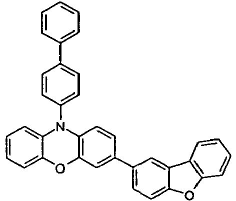 |
| 61 | 62 |
| 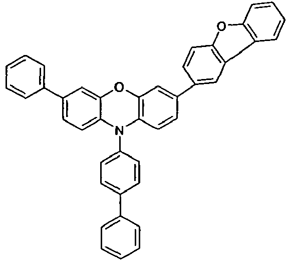 | 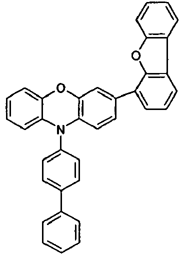 |
| 63 | 64 |
| 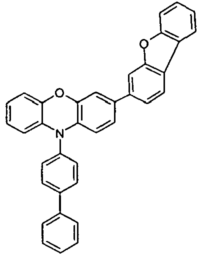 | 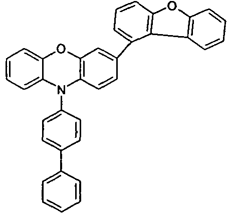 |
| 65 | 66 |
| 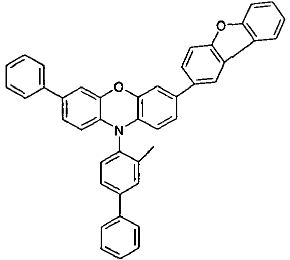 | 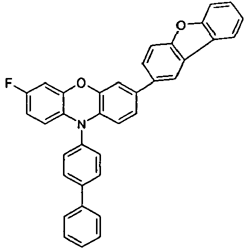 |
| 67 | 68 |
| 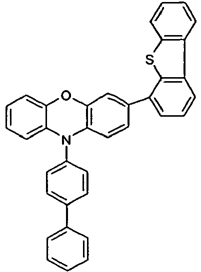 | 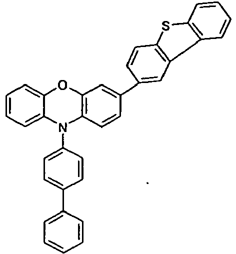 |
| 69 | 70 |
| 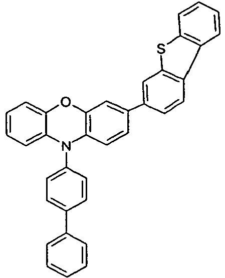 | 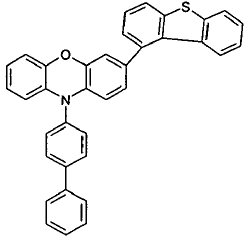 |
| 71 | 72 |
| 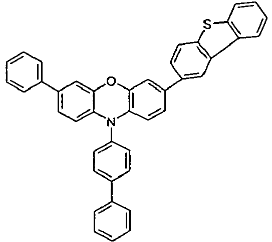 | 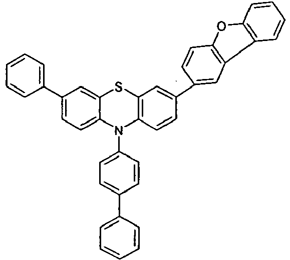 |
| 73 | 74 |
| 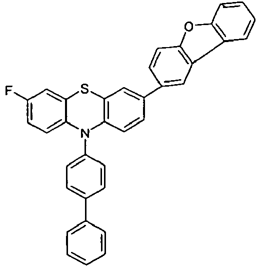 | 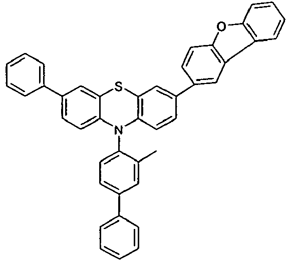 |
| 75 | 76 |
| 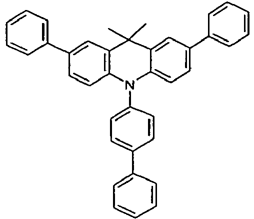 | 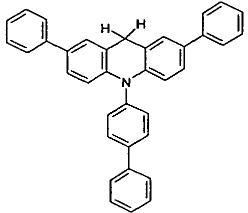 |
| 77 | 78 |
| 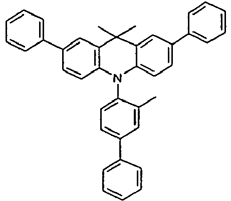 | 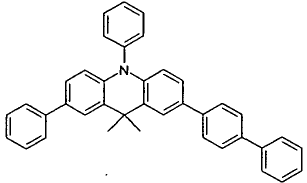 |
| 79 | 80 |
| 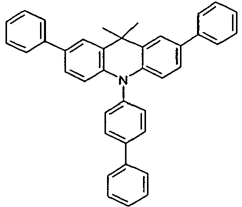 | 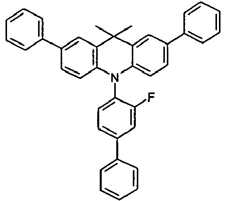 |
| 81 | 82 |
| 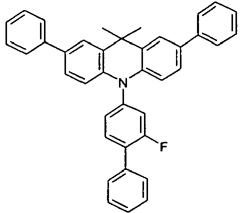 | 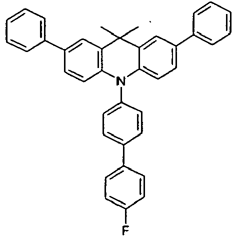 |
| 83 | 84 |
| 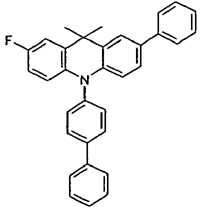 | 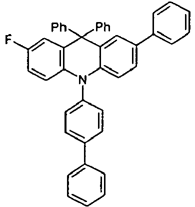 |
| 85 | 86 |
| 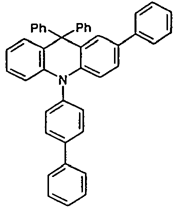 | 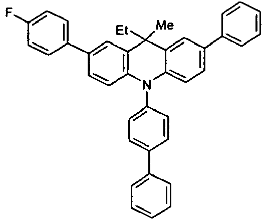 |
| 87 | 88 |
| 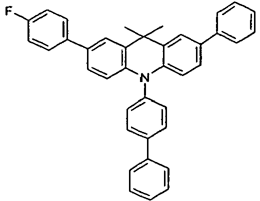 | 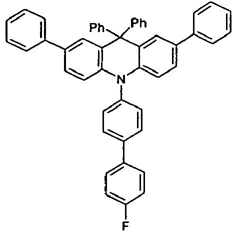 |
| 89 | 90 |
| 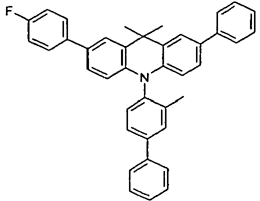 | 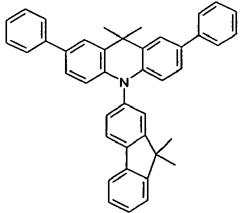 |
| 91 | 92 |
| 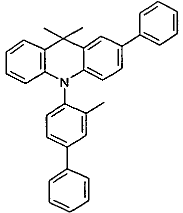 | 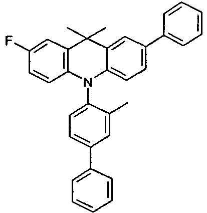 |
| 93 | 94 |
| 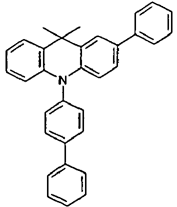 | 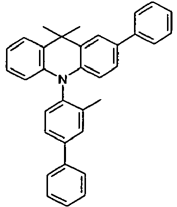 |
| 95 | 96 |
| 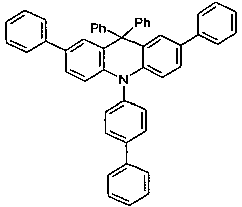 | 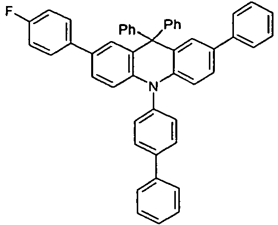 |
| 97 | 98 |
| 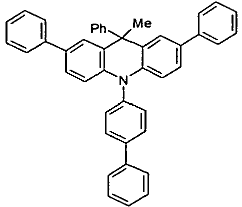 | 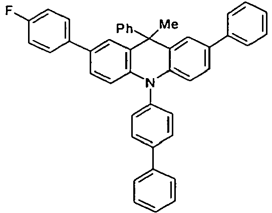 |
| 99 | 100 |
| 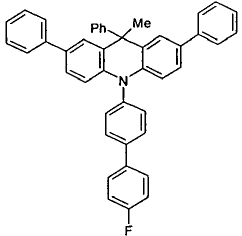 | 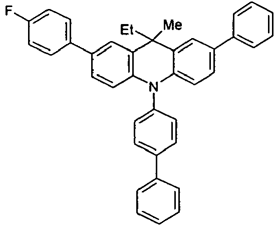 |
| 101 | 102 |
| 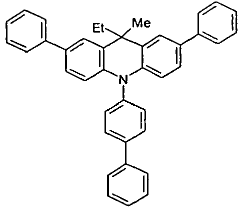 | 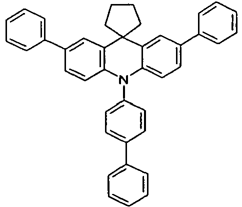 |
| 103 | 104 |
| 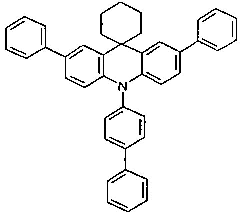 | 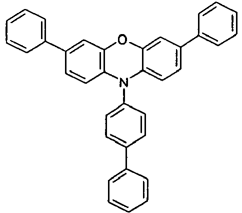 |
| 105 | 106 |
| 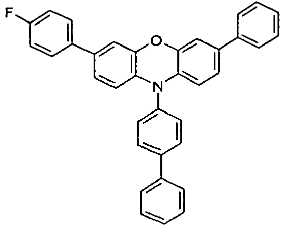 | 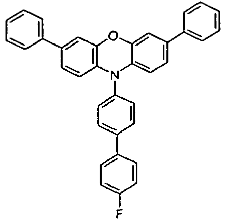 |
| 107 | 108 |
| 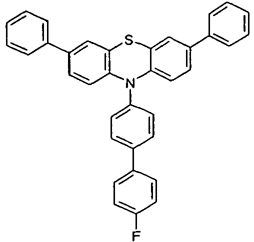 | 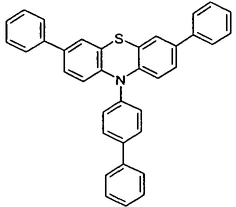 |
| 109 | 110 |
| 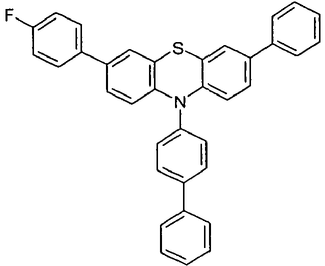 | 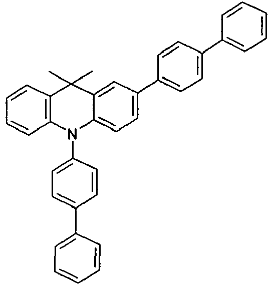 |
| 111 | 112 |
| 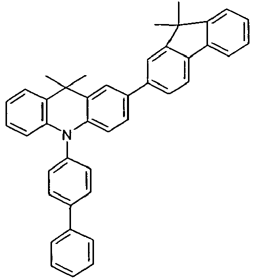 | 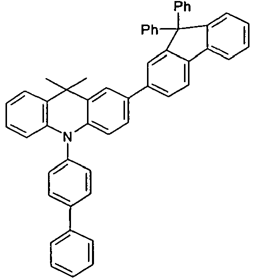 |
| 113 | 114 |
| 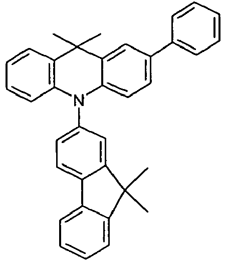 | 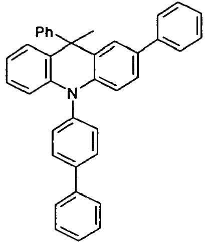 |
| 115 | 116 |
| 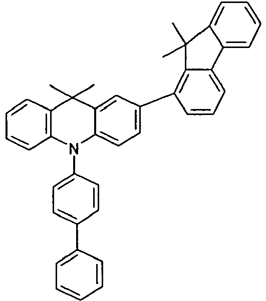 | 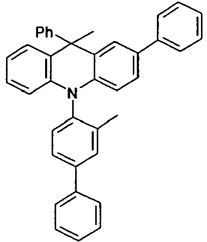 |
| 117 | 118 |
| 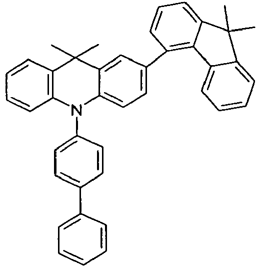 | 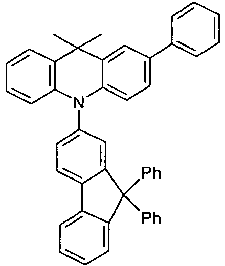 |
| 119 | 120 |
| 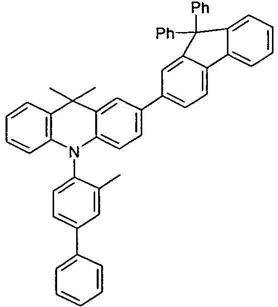 | 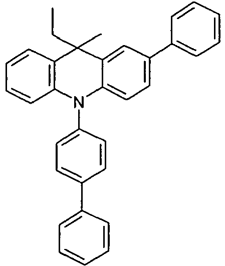 |
| 121 | 122 |
| 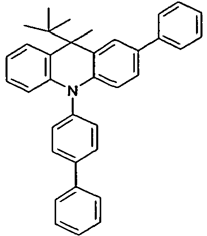 | 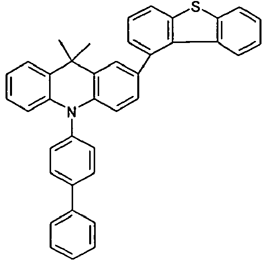 |
| 123 | 124 |
| 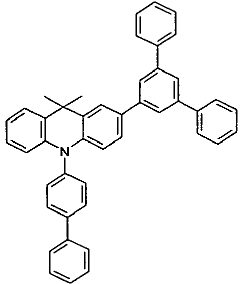 | 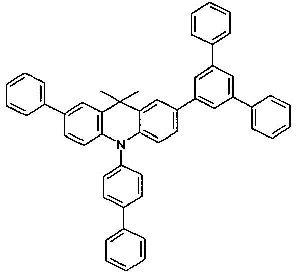 |
| 125 | 126 |
| 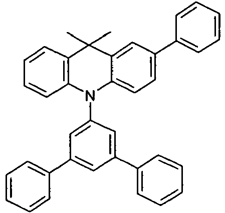 | 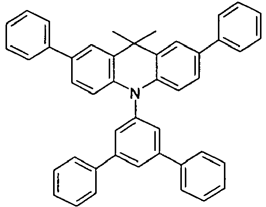 |
| 127 | 128 |
| 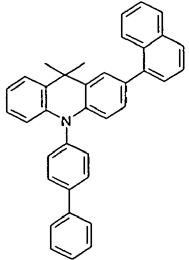 | 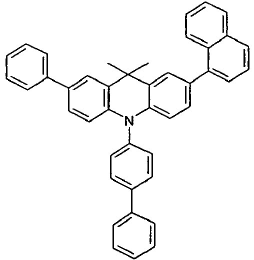 |
| 129 | 130 |
| 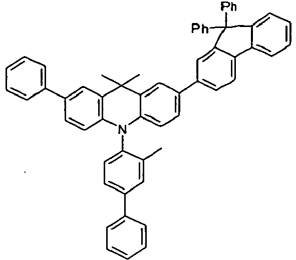 | 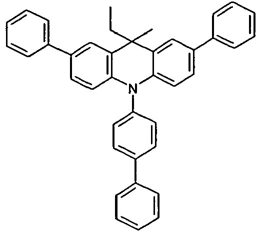 |
| 131 | 132 |
| 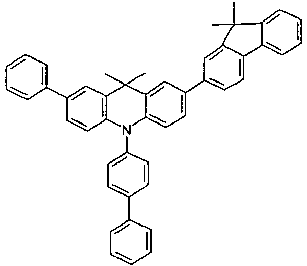 | 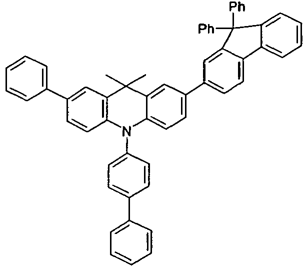 |
| 133 | 134 |
| 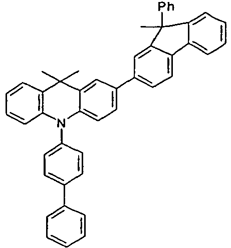 | 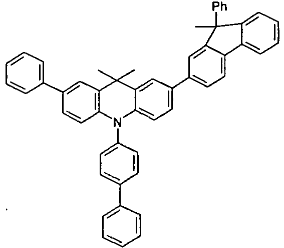 |
| 135 | 136 |
| 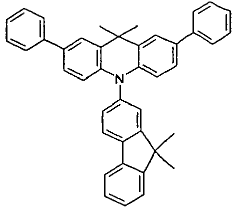 | 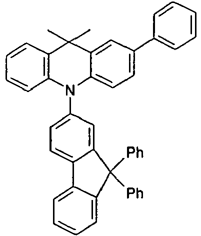 |
| 137 | 138 |
| 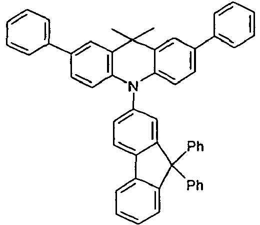 | 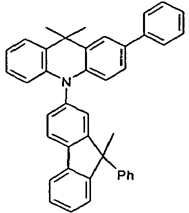 |
| 139 | 140 |
| 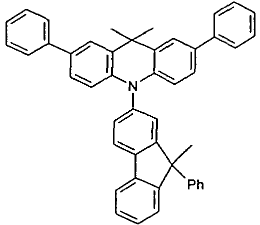 | 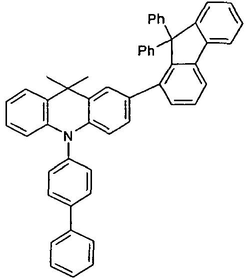 |
| 141 | 142 |
| 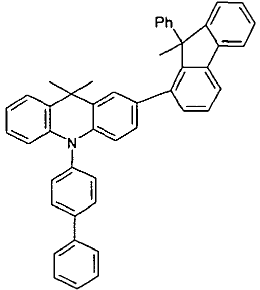 | 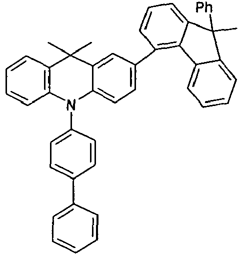 |
| 143 | 144 |
| 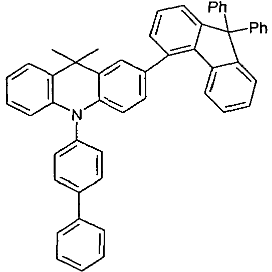 | |
| 145 | |

Die Synthese der erfindungsgemäßen Verbindungen kann gemäß dem Fachmann allgemein bekannten Verfahren der organischen präparativen Chemie erfolgen. Beispiele für bevorzugt eingesetzte Reaktionen sind Halogenierungen sowie übergangsmetallkatalysierte Kupplungsreaktionen, bevorzugt Suzuki-Kupplungen und Buchwald-Kupplungen.

Ein bevorzugtes Verfahren zur Herstellung der erfindungsgemäßen Verbindungen geht von den in Schema 1 als Edukte abgebildeten Grundstrukturen aus. Diese sind in einigen Fällen kommerziell erhältlich, in anderen Fällen können sie in wenigen Syntheseschritten aus einfachen, kommerziell erhältlichen Verbindungen hergestellt werden.

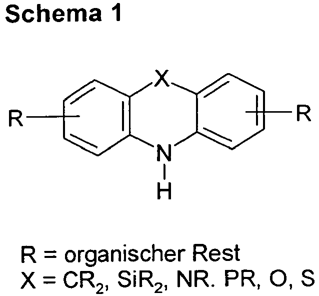

Die Verbindungen gemäß Schema 1 können bereits eine Halogengruppe oder eine andere reaktive Abgangsgruppe enthalten.

Die Verbindungen werden zunächst in einer Buchwald-Kupplung mit einer Aryl- oder Heteroaryl-Verbindung Ar-Y umgesetzt, wodurch der Substituent am Stickstoffatom eingeführt wird (Schema 2).

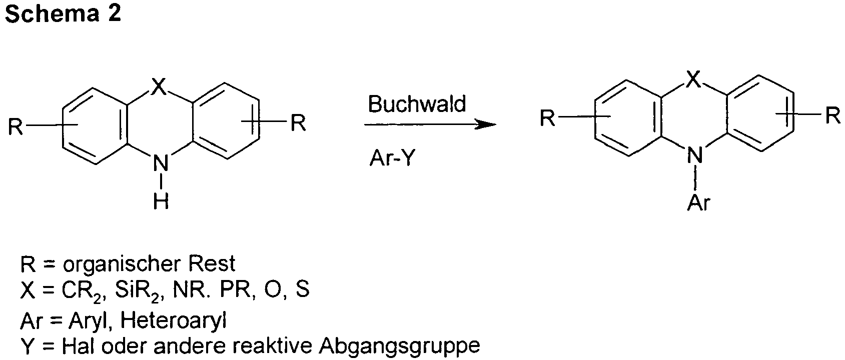

Anschließend erfolgt eine Halogenierungsreaktion an der Dihydroacridin-Einheit, soweit in der Verbindung nicht bereits eine Halogen- oder sonstige reaktive Gruppe an dieser Einheit vorhanden ist (Schema 3).

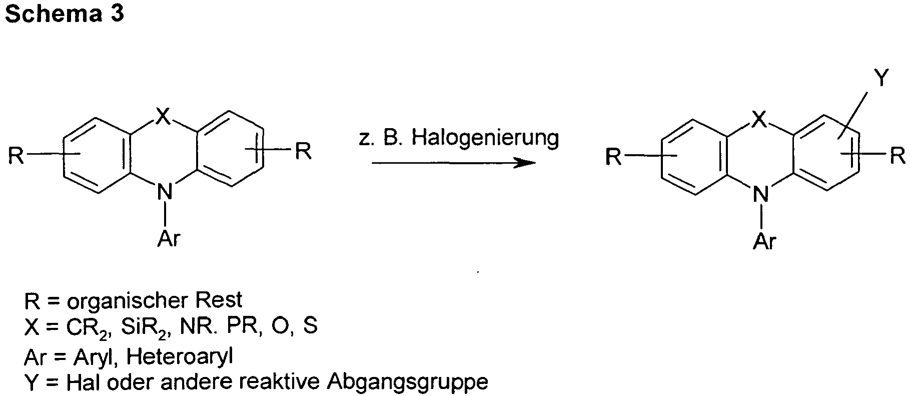

Statt einer einzigen Halogen- oder sonstigen Abgangsgruppe können auch zwei oder mehr solcher Gruppen eingeführt werden.

Abschließend wird über eine Suzuki-Kupplung an der Position der Halogen- oder sonstigen Abgangsgruppe eine weitere Aryl- oder Heteroarylgruppe in die Verbindung eingeführt (Schema 4).

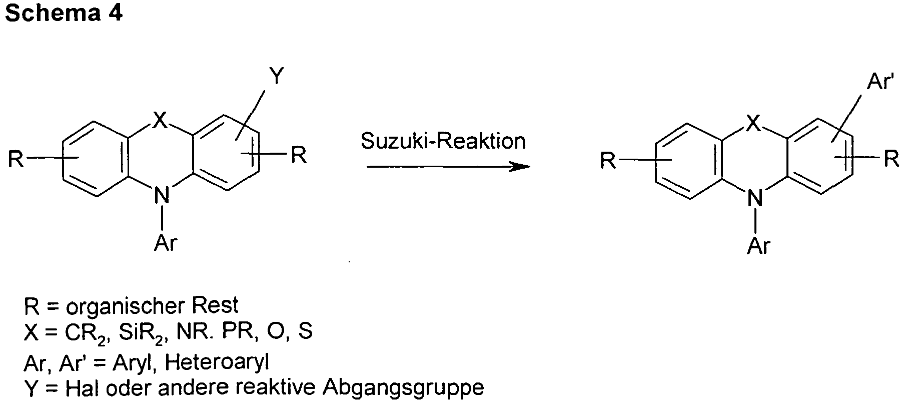

Das oben gezeigte Syntheseverfahren hat examplarischen Charakter und kann vom Fachmann auf dem Gebiet der organischen Synthese in geeigneter Weise abgewandelt werden, wenn dies für die Synthese bestimmter Ausführungsformen von erfindungsgemäßen Verbindungen vorteilhaft ist.

Einen weiteren Gegenstand der vorliegenden Erfindung stellt also ein Verfahren zur Herstellung von Verbindungen gemäß Formel (I) dar, welches dadurch gekennzeichnet ist, dass ausgehend von einem Dihydroacridin-Derivat eine oder mehrere übergangsmetallkatalysierte Kupplungsreaktionen durchgeführt werden, mit denen Aryl- oder Heteroarylgruppen als Substituenten eingeführt werden. Bevorzugt sind die übergangsmetallkatalysierten Kupplungsreaktionen ausgewählt aus Hartwig-Buchwald-Kupplungen und Suzuki-Kupplungen.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Gegenstand der Erfindung ist daher weiterhin eine Formulierung, insbesondere eine Lösung, Dispersion oder Miniemulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weitere Gegenstände der Erfindung sind daher die Verwendung der Verbindungen gemäß Formel (I) in elektronischen Vorrichtungen sowie elektronische Vorrichtungen selbst, welche eine oder mehrere Verbindungen gemäß Formel (I) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung sind, wie bereits oben ausgeführt, elektronische Vorrichtungen, enthaltend mindestens eine Verbindung gemäß Formel (I). Dabei sind die elektronischen Vorrichtungen bevorzugt ausgewählt aus den oben genannten Vorrichtungen. Besonders bevorzugt sind organische Elektrolumineszenzvorrichtungen (OLEDs), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (I) in einer elektronischen Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (I) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen.

Explizite Beispiele für geeignete phosphoreszierende Emitterverbindungen können weiterhin der folgenden Tabelle entnommen werden.

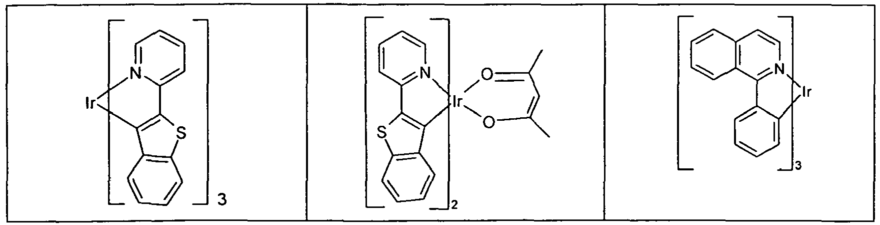

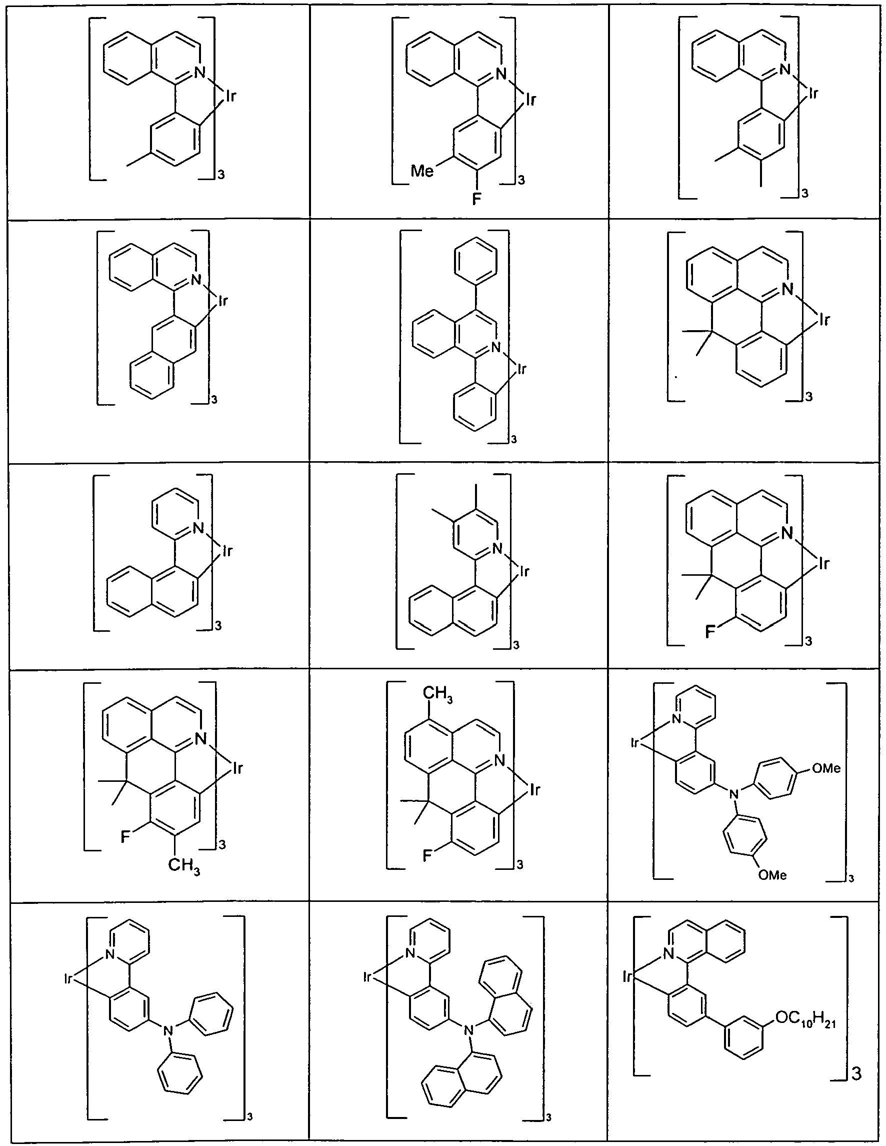

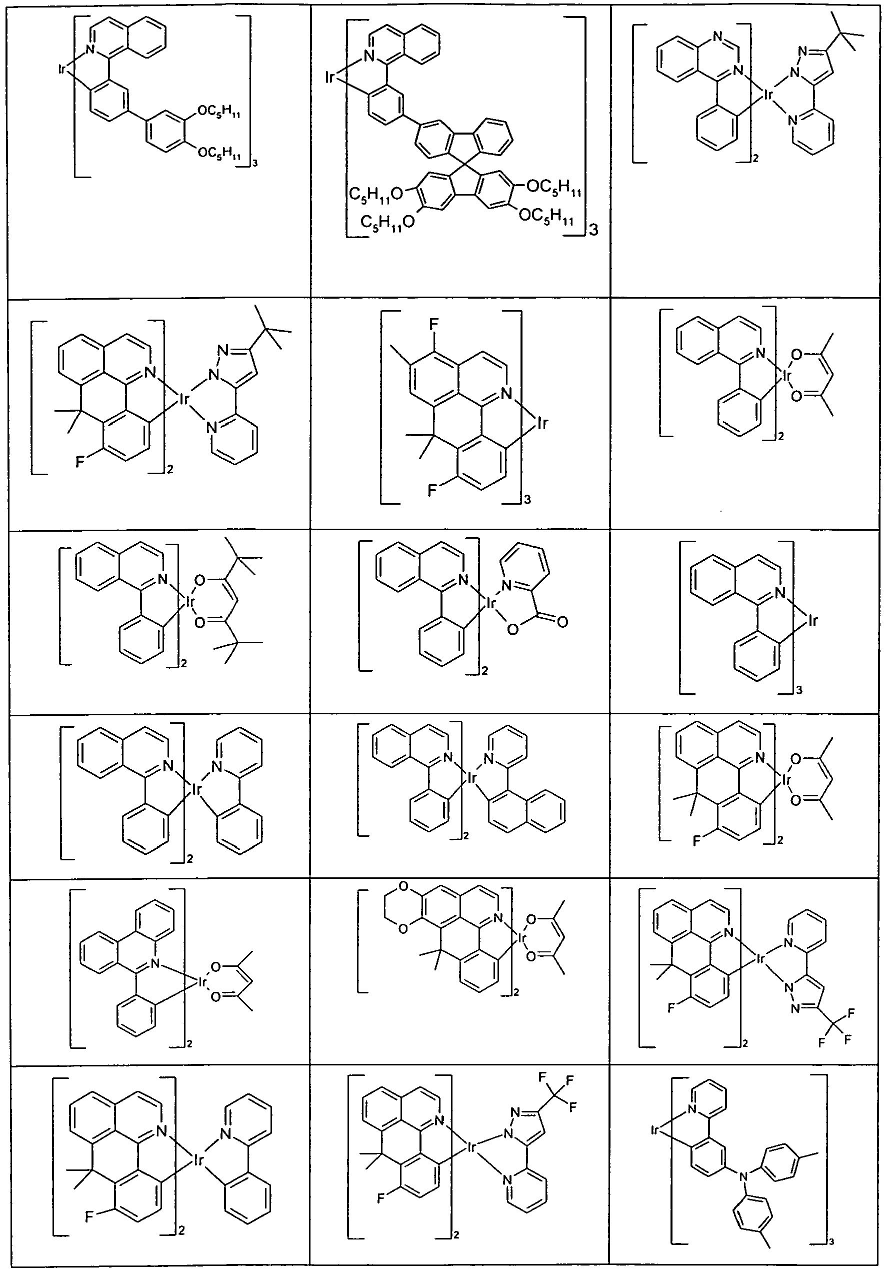

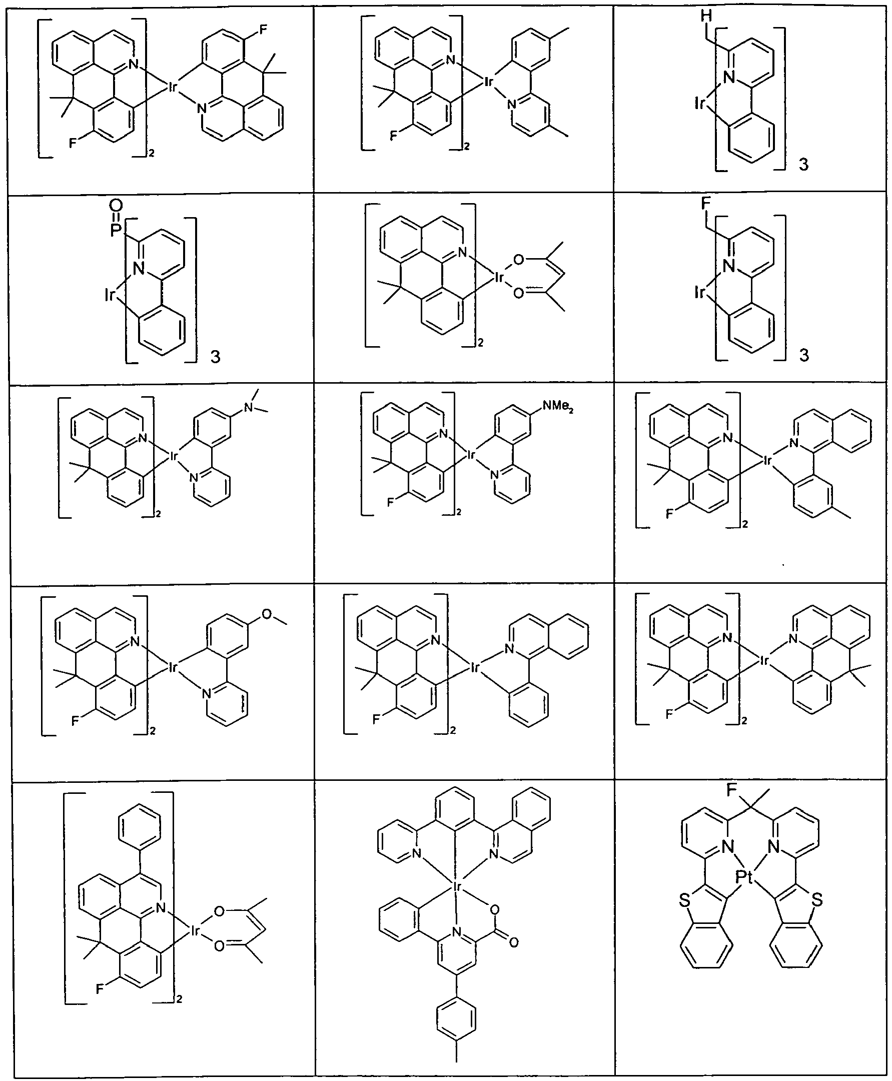

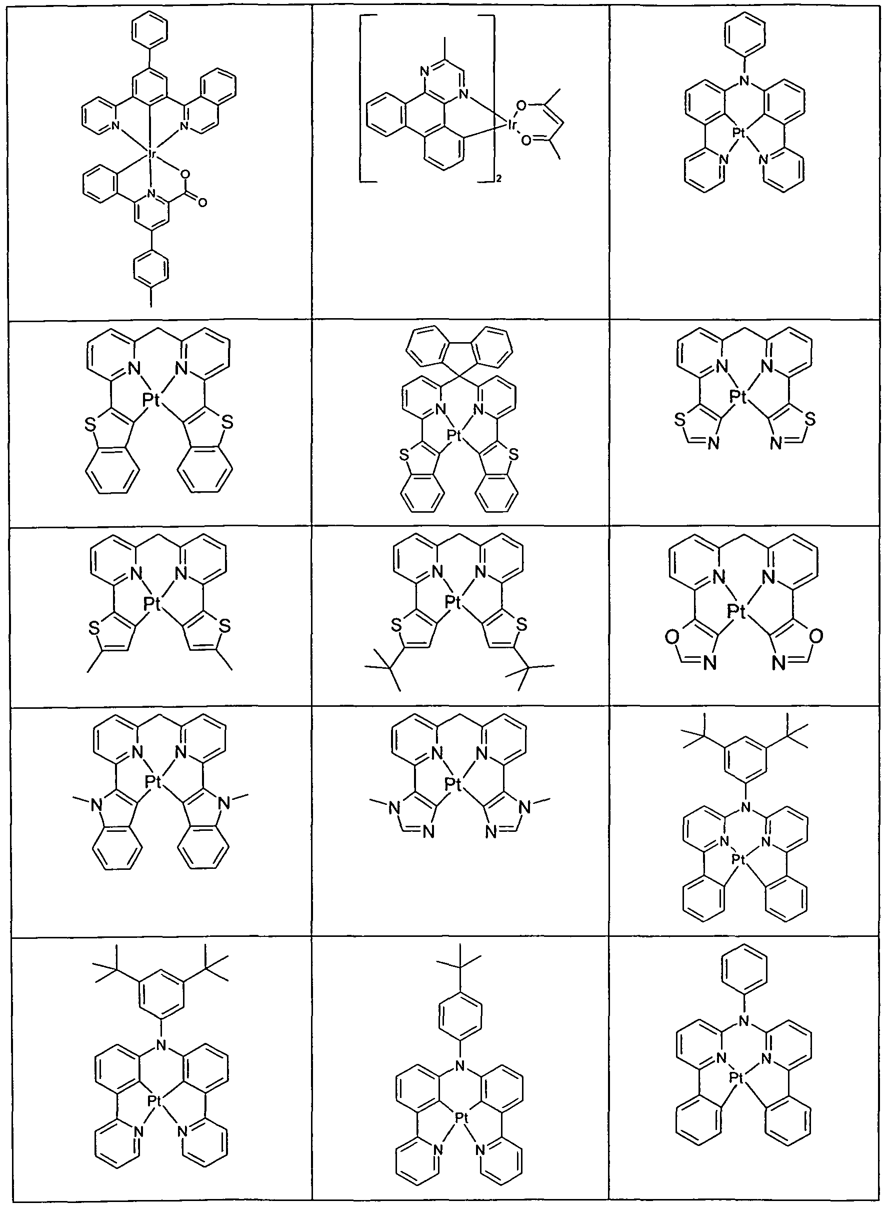

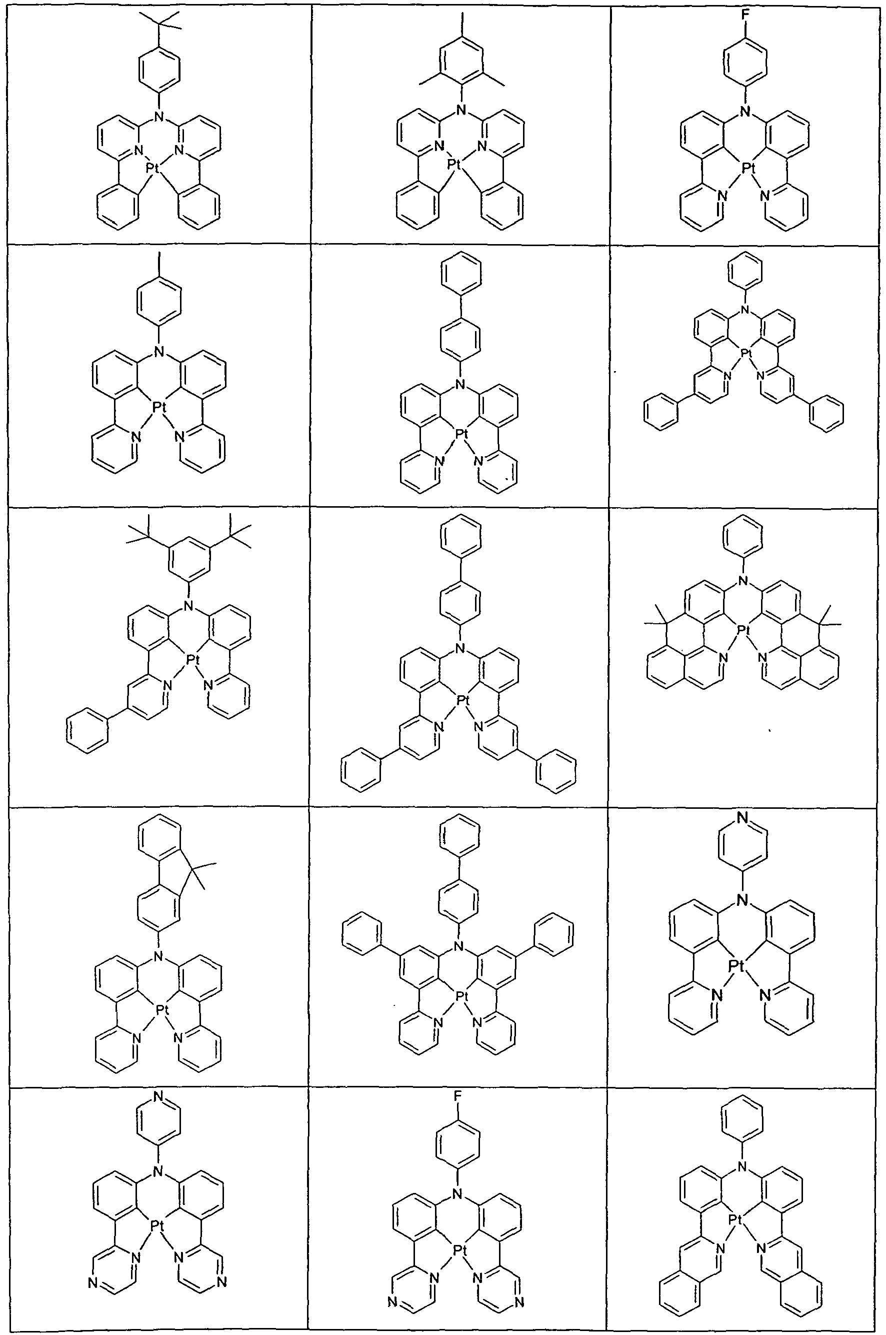

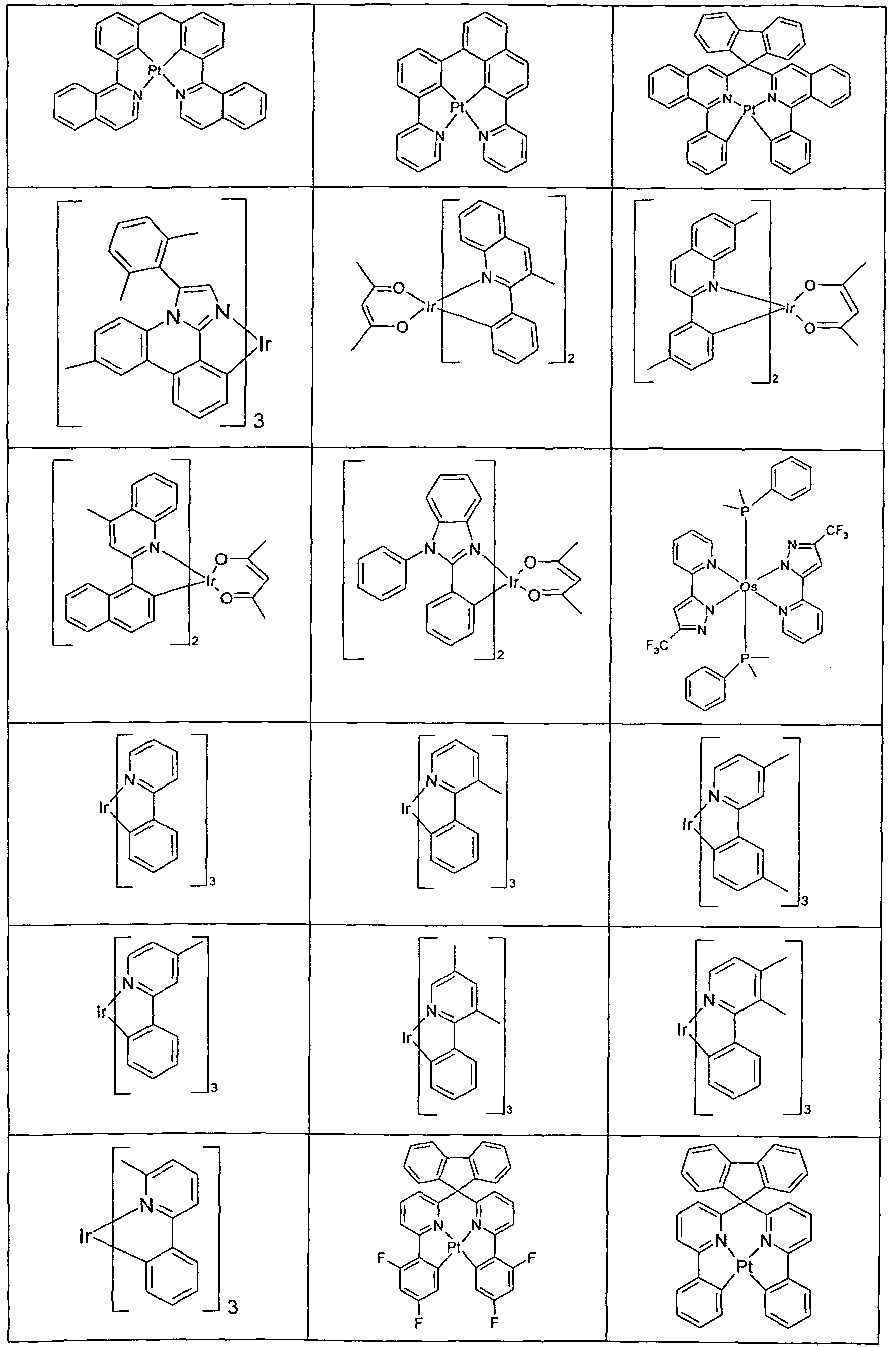

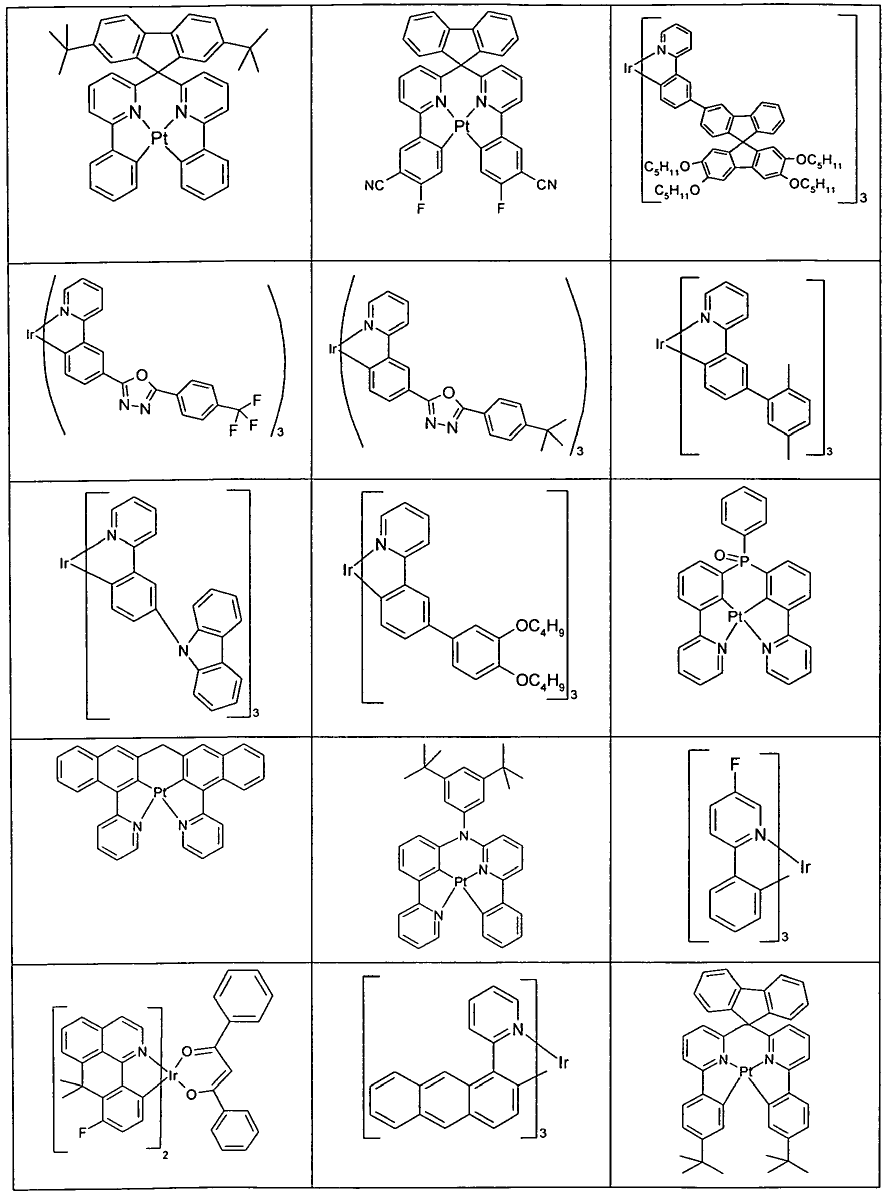

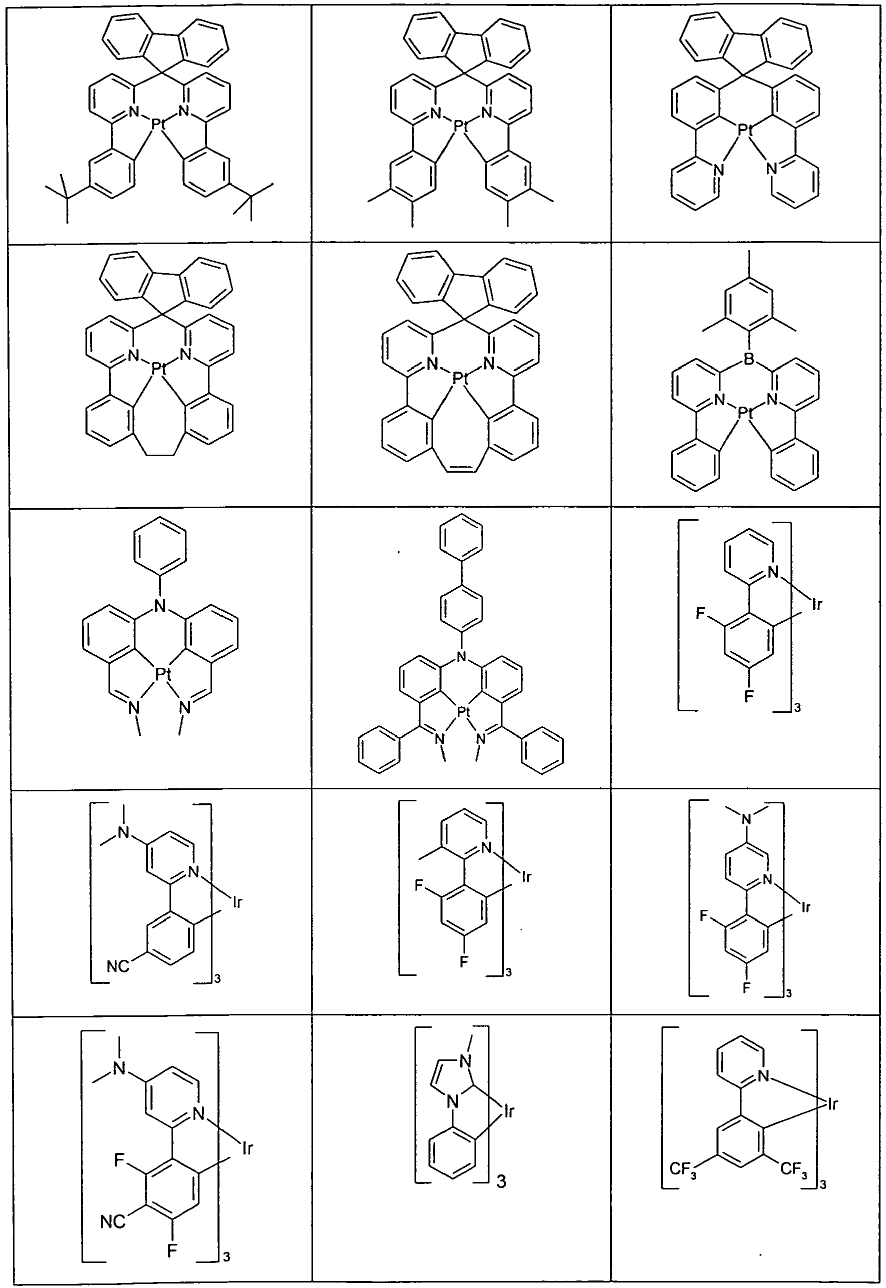

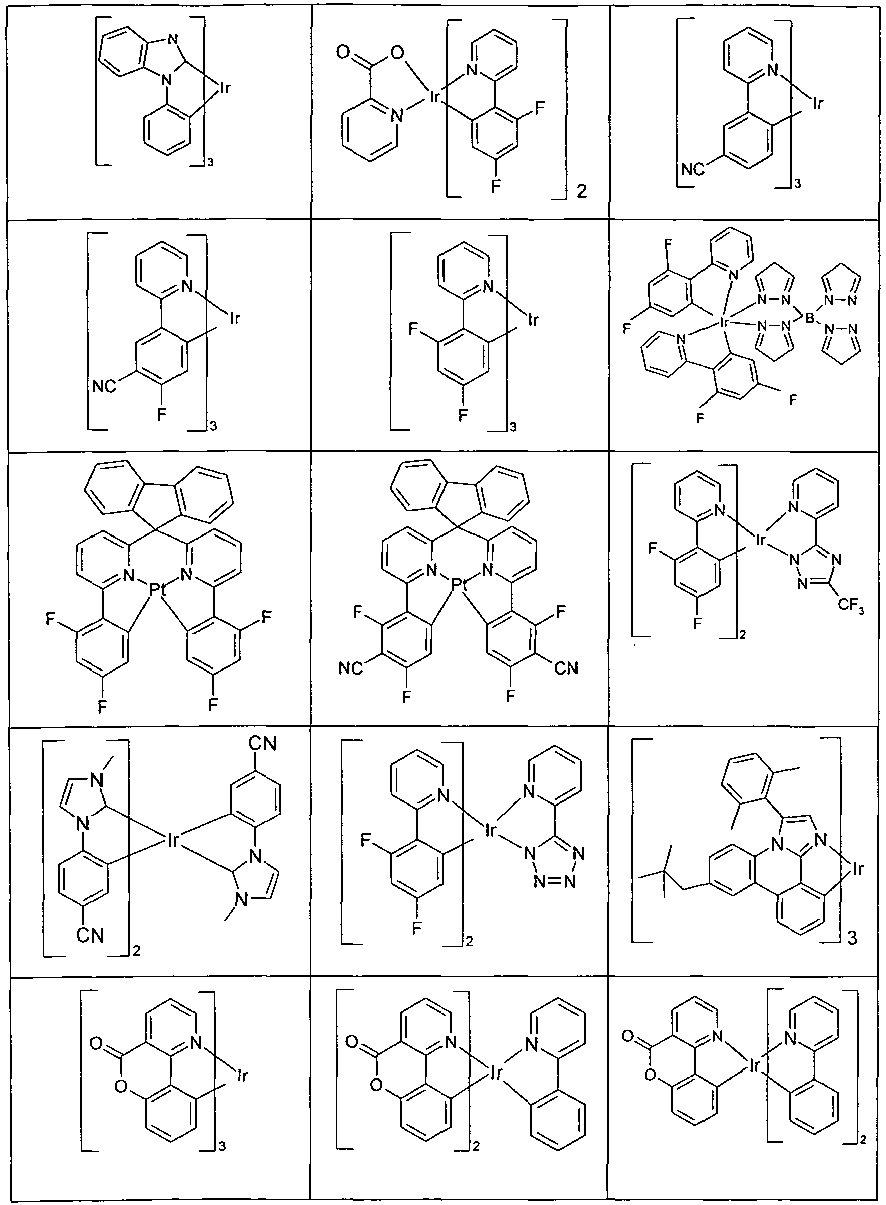

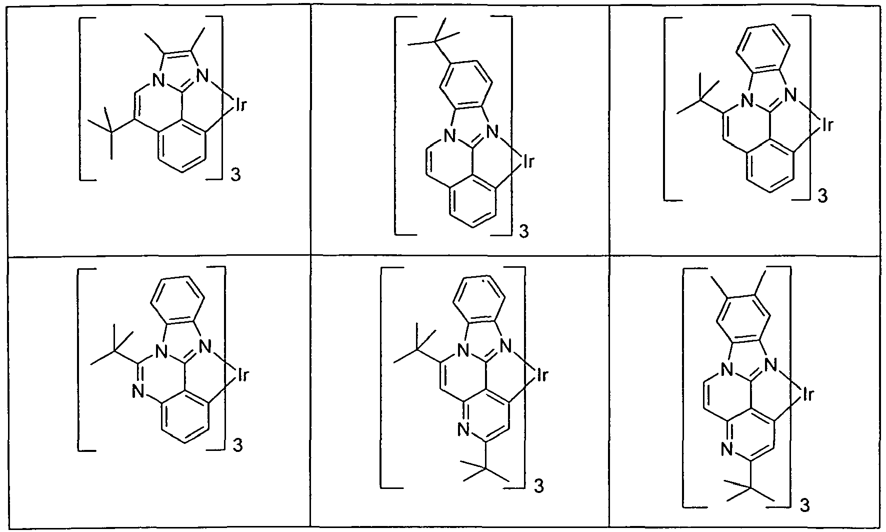

In einer bevorzugten Ausführungsform der Erfindung werden die Verbindungen gemäß Formel (I) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (I) als Lochtransportmaterial oder als Lochinjektionsmaterial verwendet werden, kann es bevorzugt sein, wenn sie mit Elektronenakzeptor-Verbindungen dotiert sind, beispielsweise mit F₄-TCNQ oder mit Verbindungen, wie sie in EP 1476881 oder EP 1596445 beschrieben werden. In einer weiteren bevorzugten Ausführungsform der Erfindung wird eine Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Wird die Verbindung gemäß Formel (I) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der Formel (I) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAlQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransportschicht oder einer Lochinjektionsschicht einer organischen Elektrolumineszenzvorrichtung, insbesondere aufgrund ihrer hohen Lochbeweglichkeit.
2. Die erfindungsgemäßen Verbindungen weisen eine relativ niedrige Sublimationstemperatur, eine hohe Temperaturstabilität sowie eine hohe Oxidationsstabilität in Lösung auf und sind somit gut prozessierbar.
3. Die erfindungsgemäßen Verbindungen, insbesondere eingesetzt als Lochtransport- oder Lochinjektionsmaterial, führen zu hohen Effizienzen sowie zu langen Lebensdauern der organischen Elektrolumineszenzvorrichtungen.

Die Erfindung wird durch die nachfolgenden Anwendungsbeispiele näher erläutert, wobei die Erfindung nicht auf den Umfang der Beispiele beschränkt ist.

### Ausführungsbeispiele

### A) Synthesebeispiele

### Synthese der Vorstufe V1:

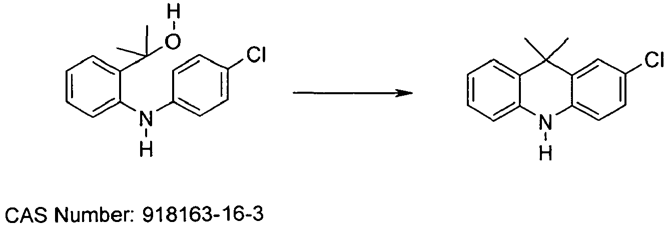

### 1. Stufe: 2-Chlor-9,9-dimethyl-9,10-dihydro-acridin

30.3 g (116 mmol) 2-[2-(4-Chlor-phenylamino)-phenyl]-propan-2-ol wurden in 700 mL entgastem Toluol gelöst und mit einer Suspension aus 93 g Polyphosphorsäure und 61.7 g Methansulfonsäure versetzt und für 1 h bei Raumtemperatur gerührt und 1 h auf 50 °C erhitzt. Der Ansatz wurde abgekühlt und auf Eis gegeben und dreimal mit Essigsäureester extrahiert. Die vereinigten org. Phasen wurden mit ges. Natriumchloridlösung gewaschen, über Magnesiumsulfat getrocknet und eingeengt. Nach Filtration des Rohproduktes über Kieselgel mit Heptan/Essigsäureester (20:1) erhielt man 25.1 g (89 %) 2-Chlor-9,9-dimethyl-9,10-dihydro-acridin als hellgelbe Kristalle.

### 2. Stufe: 10-Biphenyl-4-yl-2-Chlor-9,9-dimethyl-9,10-dihydro-acridin

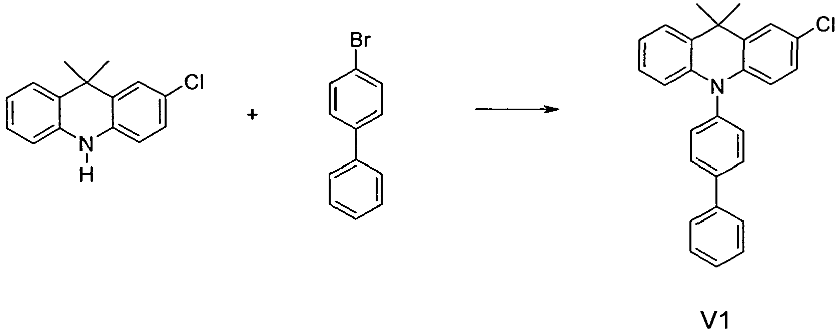

Eine entgaste Lösung von 57.9 g (243.7 mmol) 4-Brombiphenyl und 50 g (203.1 mmol) 2-Chlor-9,9-dimethyl-9,10-dihydro-acridin in 1000 mL Toluol wurde 1 h mit N₂ gesättigt. Danach wurde die Lösung zuerst mit 5.6 g (10.1mmol) DPPF, dann mit 2.28 g (10.1 mmol) Palladium(II)acetat versetzt und anschließend wurde 52.3 g (528 mmol) NaO*t*Bu im festen Zustand zugegeben. Die Reaktionsmischung wurde über Nacht unter Rückfluss erhitzt. Nach Abkühlen auf Raumtemperatur wurden vorsichtig 500 mL Wasser zugesetzt. Die wässrige Phase wurde mit 3x 50 mL Toluol gewaschen, über MgSO₄ getrocknet und das Lösungsmittel im Vakuum entfernt. Nach Filtration des Rohproduktes über Kieselgel mit Heptan/Essigsäureester (20:1) erhielt man 60 g (75 %) 10-Biphenyl-4-yl-2-Chlor-9,9-dimethyl-9,10-dihydro-acridin als hellgelbe Kristalle.

Weiterhin können die folgenden Verbindungen nach ähnlichen Bedingungen wie für die 2. Stufe der Verbindung V1 hergestellt werden:

| | **Edukt 1** | **Edukt 2** | **Produkt** | **Ausbeute** |
|---|---|---|---|---|
| V2 | 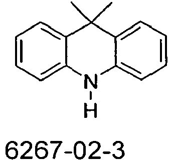 | 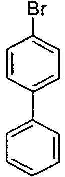 | 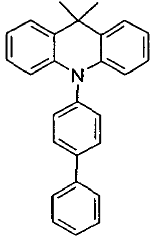 | 78% |
| V3 | 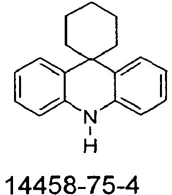 | 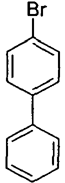 | 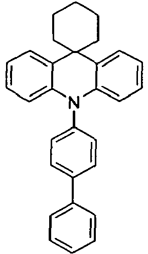 | 92% |
| V4 | 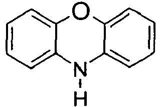 | 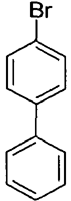 | 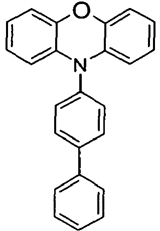 | 88% |
| V5 | 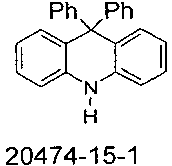 | 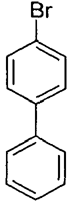 | 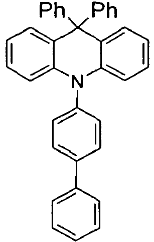 | 85% |
| V6 | 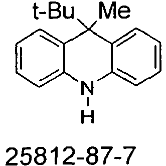 | 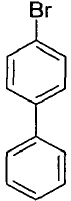 | 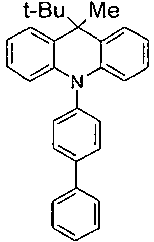 | 80% |
| V7 | 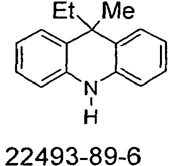 | 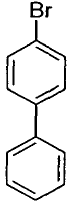 | 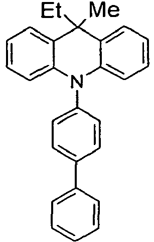 | 88% |
| V8 | 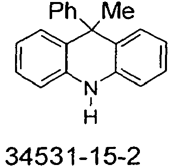 | 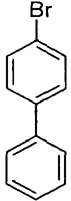 | 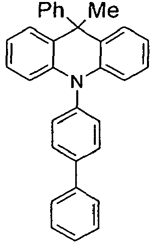 | 81% |
| V9 | 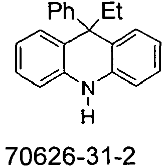 | 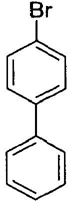 | 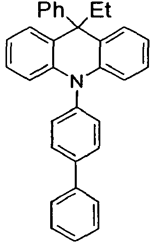 | 75% |

Aus den Verbindungen V2-V9 können durch Halogenierung die Verbindungen V2a bis V9a hergestellt werden:

### 10-Biphenyl-4-yl-2,7-dibromo-9,9-dimethyl-9,10-dihydro-acridin

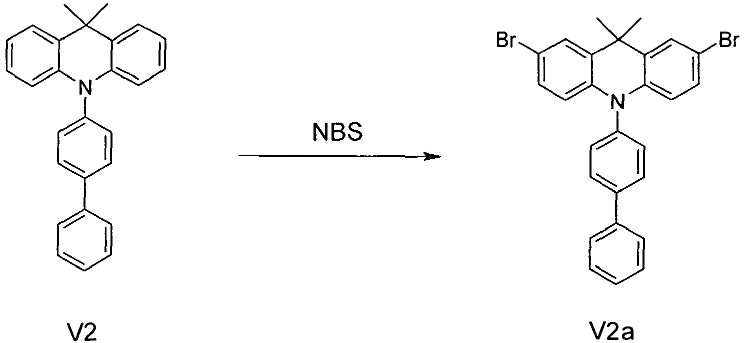

Eine Lösung des Dihydroacridins (9.8 g, 55.3 mmol) in Dichlormethan (140 mL) wurde bei 0 °C unter Lichtausschluss portionsweise mit *N-*Bromsuccinimid (9.8 g, 55.3 mmol) versetzt und 2 h bei dieser Temperatur gerührt. Die Reaktion wurde durch Zugabe von Natriumsulfit-Lösung beendet und weitere 30 min bei Raumtemperatur gerührt. Nach Phasentrennung wurde die organische Phase mit Wasser gewaschen und die wässrige Phase mit Dichlormethan extrahiert. Die vereinigten organischen Phasen wurden über Natriumsulfat getrocknet und im Vakuum eingeengt. Der Rückstand wurde in Essigsäureethylester gelöst und über Kieselgel filtriert. Anschließend wurde das Rohprodukt aus Heptan umkristallisiert. Ausbeute: 14 g, 97 % d. Th., farbloser Feststoff.

| | **Edukt 1** | **Edukt 2** | **Produkt** | **Ausbeute** |
|---|---|---|---|---|
| V3a | 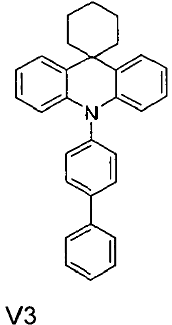 | 1 eq. NBS | 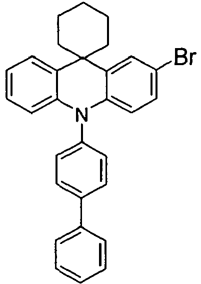 | 92% |
| V4a | 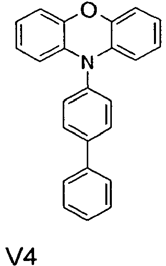 | 1 eq. NBS | 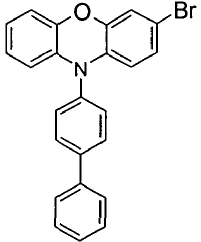 | 97% |
| V5a | 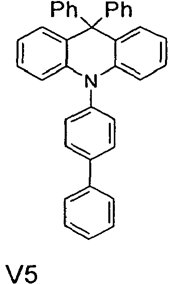 | 1 eq.NBS | 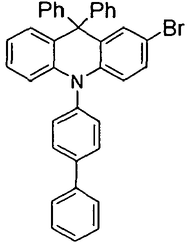 | 95% |
| V5b | 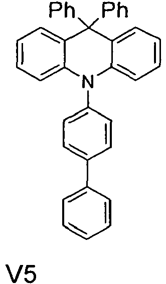 | 2 eq. NBS | 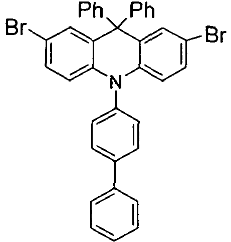 | 90% |
| V6a | 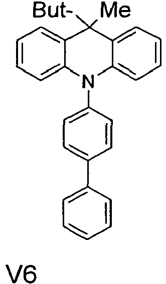 | 1 eq. NBS | 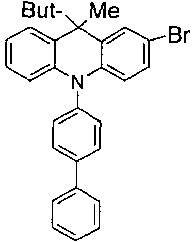 | 90% |
| V7a | 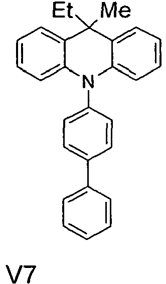 | 1 eq. NBS | 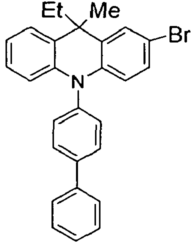 | 90% |
| V8a | 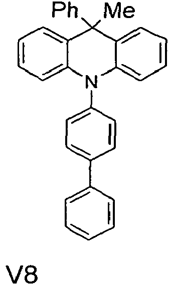 | 1 eq. NBS | 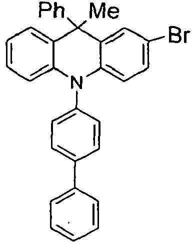 | 90% |
| V9a | 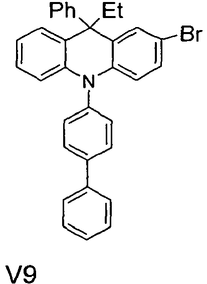 | 1 eq. NBS | 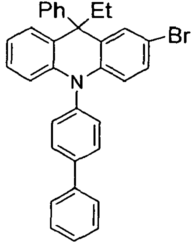 | 90% |

Durch Suzuki-Kupplung können aus den Zwischenstufen V1, V2a, V3a, V4a, V5a und V5b die erfindungsgemäßen Verbindungen 1-14 erhalten werden.

### 10-Biphenyl-4-yl-9,9-dimethyl-2-phenyl-9,10-dihydro-acridin

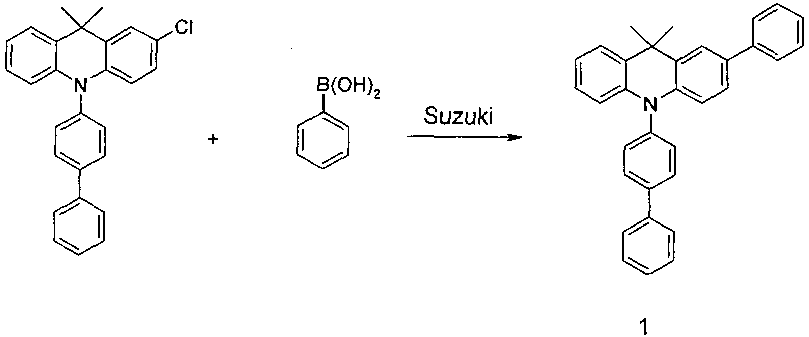

6.8 g (55.5 mmol) Benzolboronsäure, 20 g (50.5 mmol) 10-Biphenyl-4-yl-2-chlor-9,9-dimethyl-9,10-dihydro-acridin und 15.3 g (101 mmol) CsF wurden in 160 mL Dioxan suspendiert. Zu dieser Suspension wurden 1.8 g (2.5 mmol) PdCl₂(PCy₃)₂ gegeben, und die Reaktionsmischung wurde 16 h unter Rückfluss erhitzt. Nach Erkalten wurde die organische Phase abgetrennt, über Kieselgel filtriert, dreimal mit 200 mL Wasser gewaschen und anschließend zur Trockene eingeengt. Der Rückstand wurde aus Toluol umkristallisiert und abschließend im Hochvakuum sublimiert. Die Reinheit betrug 99.9 %. Die Ausbeute betrug 6.5 g entsprechend 30 % der Theorie.

Auf analogem Weg können die erfindungsgemäßen Verbindungen 2 bis 14 erhalten werden:

| | **Edukt 1** | **Edukt 2** | **Produkt** | **Ausbeute** |
|---|---|---|---|---|
| 2 | 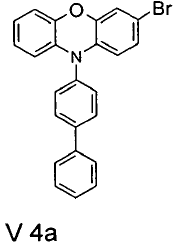 | 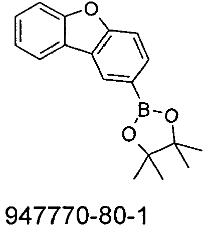 | 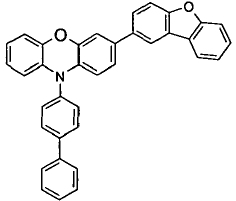 | 72 % |
| 3 | 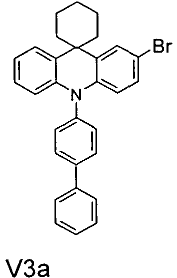 | 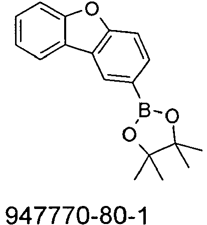 | 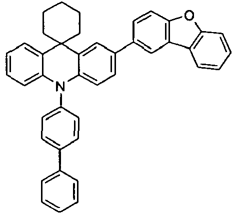 | 65% |
| 4 | 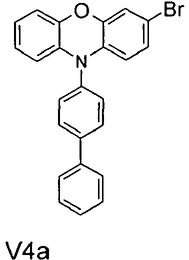 | 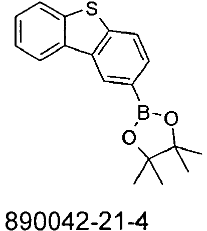 | 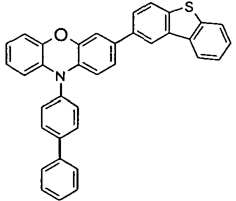 | 54% |
| 5 | 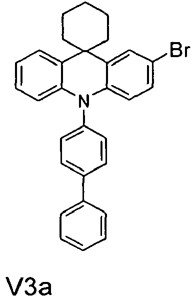 | 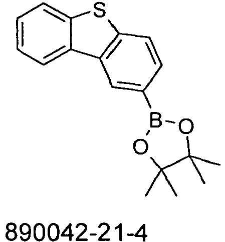 | 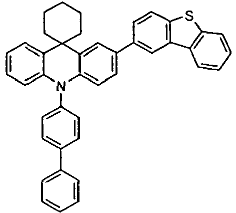 | 60% |
| 6 | 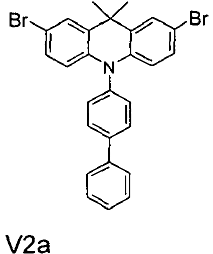 | 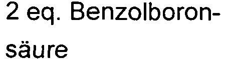 | 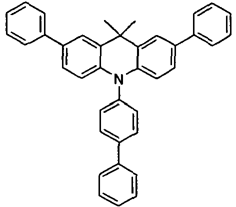 | 50% |
| 7 | 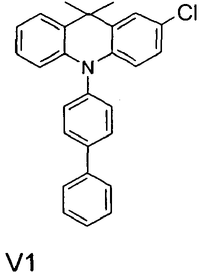 | 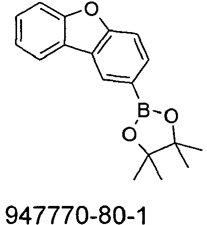 | 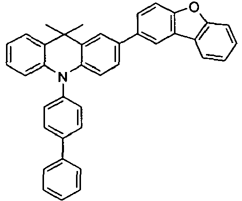 | 52% |
| 8 | 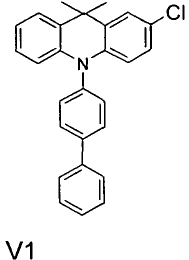 | 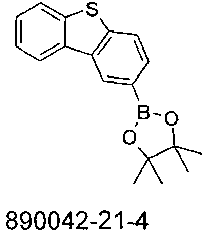 | 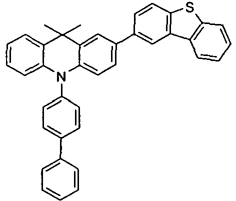 | 47% |
| 9 | 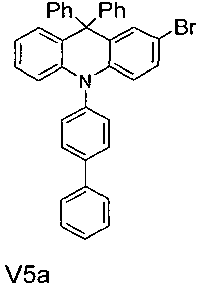 | 1 eq. Benzolboronsäure | 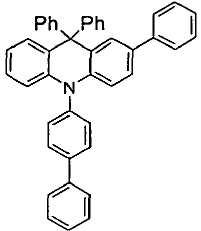 | 55% |
| 10 | 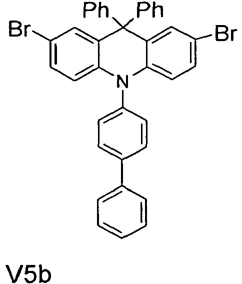 | 2.eq. Benzolboronsäure | 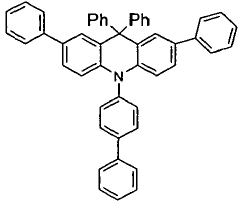 | 60% |
| 11 | 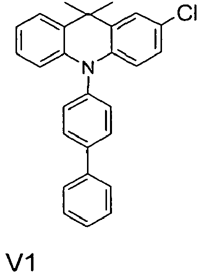 | 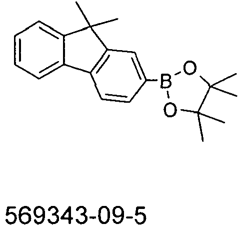 | 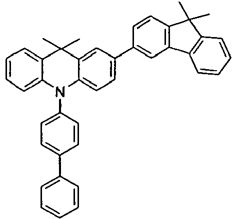 | 63% |
| 12 | 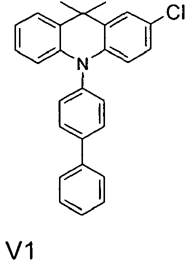 | 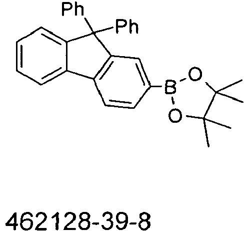 | 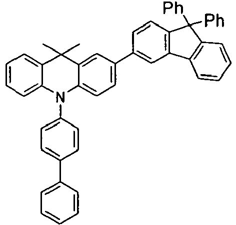 | 57% |
| 13 | 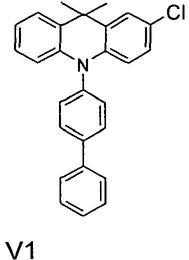 | 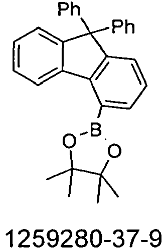 | 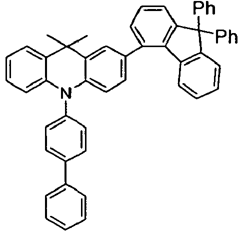 | 67% |
| 14 | 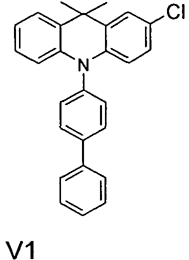 | 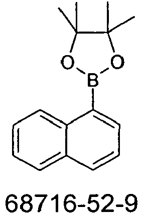 | 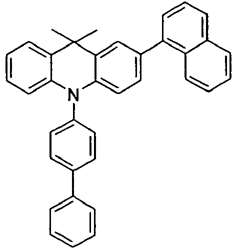 | 70% |

Weiterhin wurden aus den Vorstufen V6a bis V9a durch Suzuki-Kupplung mit Phenylboronsäure unter analogen Bedingungen die Verbindungen 15 bis 18 erhalten:

| | | |
|---|---|---|
| 15 | 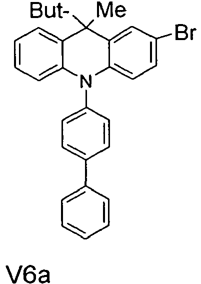 | 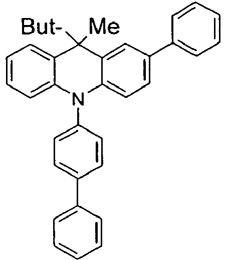 |
| 16 | 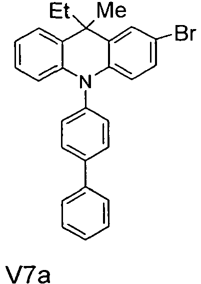 | 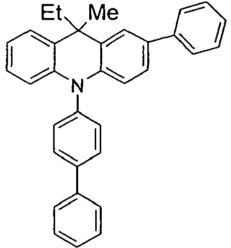 |
| 17 | 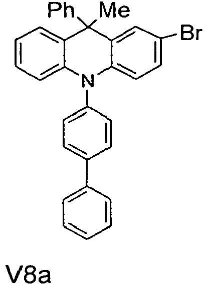 | 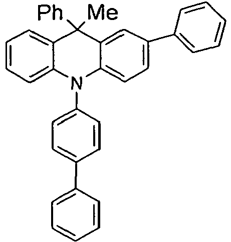 |
| 18 | 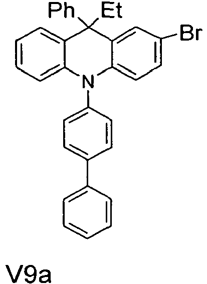 | 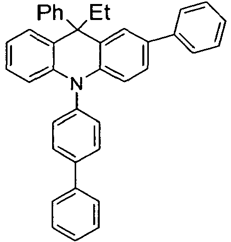 |

### B) Device-Beispiele

### Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen V1 bis V8 und E1 bis E6 (siehe Tabellen 1 bis 5) werden der Aufbau sowie die Daten verschiedener OLEDs vorgestellt. Glasplättchen, die mit strukturiertem ITO (Indium Zinn Oxid) der Dicke 150 nm beschichtet sind werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / Optionale Lochinjektionsschicht (HIL) / Lochtransportschichten (HTL) / Zwischenschicht (IL) / Elektronenblockerschicht (EBL) / Emissionsschicht (EML) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist den Tabellen 1 und 3 zu entnehmen. Die zur Herstellung der OLEDs benötigten Materialien sind in Tabelle 5 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U @ 1000 cd/m² in Tabelle 2 und 4 bezeichnet die Spannung, die für eine Leuchtdichte von 1000 cd/m² benötigt wird. EQE @ 1000 cd/m² schließlich bezeichnet die externe Quanteneffizienz bei einer Betriebsleuchtdichte von 1000 cd/m². LD80 @ 6000 cd/m2 ist die Lebensdauer bis das OLED bei einer Helligkeit von 6000 cd/m² auf 80 % der Anfangsintensität, also auf 4800 cd/m² abgefallen ist.

Die gemessenen Daten der verschiedenen OLEDs sind in den Tabellen 2 und 4 zusammengefasst.

### Verwendung der erfindungsgemäßen Verbindungen in fluoreszierenden und phosphoreszierenden OLEDs

Die erfindungsgemäßen Verbindungen eignen sich insbesondere als HTM (Lochtransportmaterial) oder EBM (Elektronenblockiermaterial) in OLEDs. Sie eignen sich zur Verwendung in einer Einzelschicht, aber auch als Mixed-Komponente als HTM, EBM oder als Bestandteil der emittierenden Schicht. Verglichen mit Vergleichs-Vorrichtungen gemäß dem Stand der Technik (V1 bis V8) zeigen alle Proben mit den erfindungsgemäßen Verbindungen höhere Effizienzen verbunden mit gleichen oder verbesserten Lebensdauern (E1 bis E6).

Im Vergleich zum Referenzmaterial HTMV1 (V2 und V6) zeigen die erfindungsgemäßen Verbindungen bessere Effizienzen und bessere Lebensdauern. So ist die Lebensdauer von V2 im Vergleich zu E1 bis E3 in einer blau emittierenden Vorrichtung nahezu verdoppelt und auch in der grün emittierenden Vorrichtung (V6 gegen E4 bis E6) ergibt sich noch nahezu eine Verdopplung der Lebensdauer.

Im Vergleich zum Referenzmaterial HTMV2 (V3 und V7) ergeben sich für die erfindungsgemäßen Verbindungen bessere oder gleich gute (HTM2) Lebensdauern in blau bzw. grün emittierenden Vorrichtungen bei deutlicher Verbesserung der Effizienz.

Im Vergleich zum Referenzmaterial HTMV3 (V4 und V8) ergeben sich für die erfindungsgemäßen Verbindungen deutlich bessere Effizienzen und Lebensdauern.

| | | | | | | | |
|---|---|---|---|---|---|---|---|
| **Tabelle 1: Aufbau der OLEDs** | | | | | | | |

| ***Bsp.*** | ***IL*** | ***HTL*** | ***IL*** | ***HTL2*** | ***EBL*** | ***EML*** | ***ETL*** |
|---|---|---|---|---|---|---|---|
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIL1* | *HIL2* | *HIL1* | | *NPB* | *H1(95%):SEB1(5%)* | *ETM1(50%):LiQ(50%)* |
| | *5 nm* | *140 nm* | *5 nm* | | *20 nm* | *20 nm* | *30 nm* |
| *V2* | *HIL1* | *HIL2* | *HIL1* | | *HTMV1* | *H1(95%):SEB1(5%)* | *ETM1(50%):LiQ(50%)* |
| | *5 nm* | *140 nm* | *5 nm* | | *20 nm* | *20 nm* | *30 nm* |
| *V3* | *HIL1* | *HIL2* | *HIL1* | | *HTMV2* | *H1(95%):SEB1(5%)* | *ETM1(50%):LiQ(50%)* |
| | *5 nm* | *140 nm* | *5 nm* | | *20 nm* | *20 nm* | *30 nm* |
| *V4* | *HIL1* | *HIL2* | *HIL1* | *NPB* | *HTMV3* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E1* | *HIL1* | *HIL2* | *HIL1* | *NPB* | *HTM1* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E2* | *HIL1* | *HIL2* | *HIL1* | *NPB* | *HTM2* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E3* | *HIL1* | *HIL2* | *HIL1* | *NPB* | *HTM3* | *H1(95%):SEB1(5%)* | *ETM1(50%):LiQ(50%)* |
| | *5 nm* | *130 nm* | *5 nm* | *10 nm* | 20 *nm* | *20 nm* | *30 nm* |

| **Tabelle 2: Daten der OLEDs** | | | | | |
|---|---|---|---|---|---|
| ***Bsp.*** | ***U*@ *1000 cd*/*m2*** | ***EQE** @* ***1000 cd*/*m2*** | ***LD80 @ 6000 cd*/*m2*** | ***CIE*** | |
| | *V* | % | *[h]* | *x* | y |
| *V1* | *4.7* | *4.8* | *70* | 0.14 | 0.17 |
| *V2* | *4.2* | *5.4* | *55* | 0.14 | 0.17 |
| *V3* | *4.4* | *6.0* | *90* | 0.14 | 0.16 |
| *V4* | *4.5* | *5.7* | *60* | 0.14 | 0.16 |
| *E1* | *4.2* | *7.6* | *100* | 0.14 | 0.16 |
| *E2* | *4.3* | *8.4* | *90* | 0.14 | 0.16 |
| *E3* | *4.3* | *7.3* | *135* | 0.14 | 0.16 |

| **Tabelle 3: Aufbau der OLEDs** | | | | | | |
|---|---|---|---|---|---|---|
| ***Bsp.*** | ***HTL*** | ***IL*** | ***HTL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* | *Dicke* / *nm* |
| *V5* | *HIL2* | *HIL1* | | *NPB* | *H2(88%):Irpy(12%)* | *ETM1(50%):LiQ(50%)* |
| | *70 nm* | *5 nm* | | *90 nm* | *30 nm* | *40 nm* |
| *V6* | *HIL2* | *HIL1* | | *HTMV1* | *H2(88%):Irpy(12%)* | *ETMf(50%):LiQ(50%)* |
| | *70 nm* | *5 nm* | | *90 nm* | *30 nm* | *40 nm* |
| *V7* | *HIL2* | *HIL1* | | *HTMV2* | *H2(88%):Irpy(12%)* | *ETM1(50%):LiQ(50%)* |
| | *70 nm* | *5 nm* | | *90 nm* | *30 nm* | *40 nm* |
| *V8* | *HIL2* | *HIL1* | *NPB* | *HTMV3* | *H2(88%):Irpy(12%)* | *ETM1(50%):LiQ(50%)* |
| | *70 nm* | *5 nm* | *10 nm* | *80 nm* | *30 nm* | *40 nm* |
| *E4* | *HIL2* | *HIL1* | *NPB* | *HTM1* | *H2(88%):Irpy(12%)* | *ETM1(50%):LiQ(50%)* |
| | *70 nm* | *5 nm* | *10 nm* | *80 nm* | *30 nm* | *40 nm* |
| *E5* | *HIL2* | *HIL1* | *NPB* | *HTM2* | *H2(88%):Irpy(12%)* | *ETM1(50%):LiQ(50%)* |
| | *70 nm* | *5 nm* | *10 nm* | *80 nm* | *30 nm* | *40 nm* |
| *E6* | *HIL2* | *HIL1* | *NPB* | *HTM3* | *H2(88%):Irpy(12%)* | *ETM1(50%):LiQ(50%)* |
| | *70 nm* | *5 nm* | *10 nm* | *80 nm* | *30 nm* | *40 nm* |

| **Tabelle 4: Daten der OLEDs** | | | | | |
|---|---|---|---|---|---|
| ***Bsp***. | ***U*** @ ***1000 cd*/*m2*** | ***Effizienz* @ *1000 cd*/*m2*** | ***LD80 @ 8000 cd*/*m²*** | ***CIE*** | |
| | *V* | *%* | *[h]* | *x* | *y* |
| *V5* | *3.6* | *14.4* | *85* | 0.32 | 0.63 |
| *V6* | *3.1* | *13.1* | *60* | 0.33 | 0.64 |
| *V7* | *3.2* | *17.1* | *110* | 0.33 | 0.63 |
| *V8* | *3.1* | *15.1* | 85 | 0.32 | 0.63 |
| *E4* | *3.2* | *17.6* | *140* | 0.33 | 0.64 |
| *E5* | *3.3* | *18.1* | *100* | 0.33 | 0.63 |
| *E6* | *3.1* | *17.8* | *140* | 0.33 | 0.64 |

**Tabelle 5:**

| | | |
|---|---|---|
| 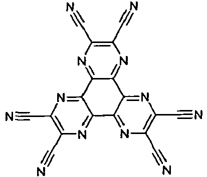 | 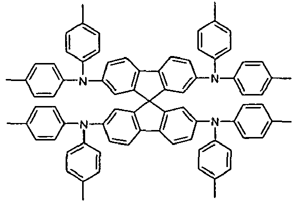 | 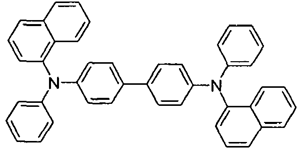 |
| HIL1 | HIL | NPB |
| 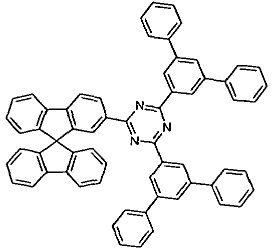 | 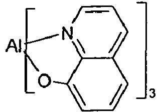 | 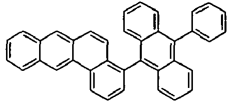 |
| ETM1 | Alq3 | H1 |
| 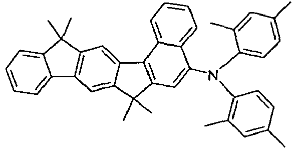 | 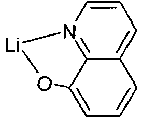 | 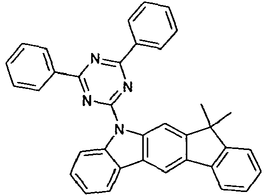 |
| SEB1 | LiQ | H2 |
| 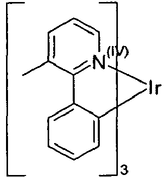 | | |
| Irpy | | |
| 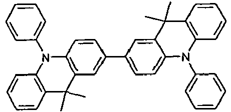 | 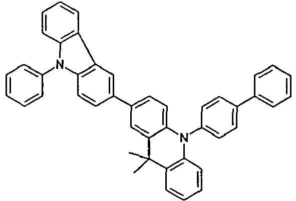 | 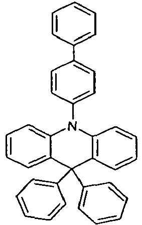 |
| HTMV1 | HTMV2 | HTMV3 |
| 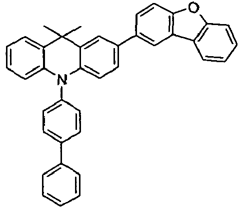 | 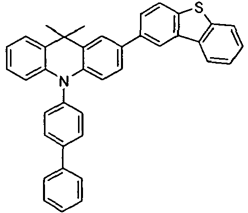 | 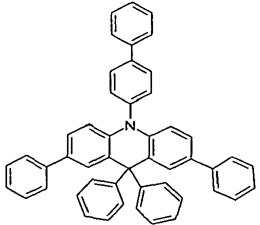 |
| HTM1 (Synthesebeispiel 7) | HTM2 (Synthesebeispiel 8) | HTM3 (Synthesebeispiel 10) |

## Patentansprüche

1. Verbindung einer Formel (I) 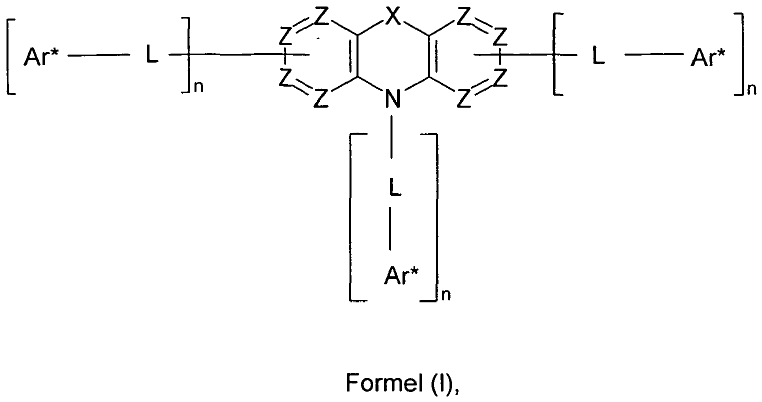 wobei für die auftretenden Symbole und Indices gilt:
Ar* ist bei jedem Auftreten gleich oder verschieden ein aromatisches Ringsystem mit 6 bis 24 aromatischen Ringatomen oder eine elektronenreiche Heteroarylgruppe mit 5 bis 18 aromatischen Ringatomen, welche jeweils mit einem oder mehreren Resten R² substituiert sein können;
L ist bei jedem Auftreten gleich oder verschieden eine Einfachbindung, C=O, C=NR², Si(R²)₂, PR², P(=O)(R²), O, S, SO, SO₂, eine Alkylengruppe mit 1 bis 20 C-Atomen oder eine Alkenylen- oder Alkinylengruppe mit 2 bis 20 C-Atomen, wobei in den genannten Gruppen eine oder mehrere CH₂-Gruppen durch C=O, C=NR², C=O-O, C=O-NR², Si(R²)₂, NR², P(=O)(R²), O, S, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 24 aromatischen Ringatomen, welches durch einen oder mehrere Reste R² substituiert sein kann, oder eine beliebige Kombination aus 1, 2, 3, 4 oder 5 gleichen oder verschiedenen Gruppen ausgewählt aus den oben genannten Gruppen;
X ist eine divalente Gruppe ausgewählt aus C(R¹)₂, Si(R¹)₂, NR¹, PR¹, O und S;
Z ist bei jedem Auftreten gleich oder verschieden CR² oder N, sofern Z keinen Substituenten trägt, und ist gleich C, sofern Z einen Substituenten trägt;
R¹ ist bei jedem Auftreten gleich oder verschieden H, D, F, Cl, Br, I, C(=O)R³, CN, Si(R³)₃, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R³ substituiert sein kann, wobei zwei oder mehr nichtaromatische Reste R¹ miteinander verknüpft sein können und einen Ring bilden können;
R² ist bei jedem Auftreten gleich oder verschieden H, D, F, Cl, Br, I, C(=O)R³, CN, Si(R³)₃, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C=C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy-oder Heteroaryloxygruppe mit 5 bis 30 aromatischen Ringatomen, die durch einen oder mehrere Reste R³ substituiert sein kann, wobei zwei oder mehr Reste R² miteinander verknüpft sein können und einen Ring bilden können;
R³ ist bei jedem Auftreten gleich oder verschieden H, D, F, Cl, Br, I, C(=O)R⁴, CN, Si(R⁴)₃, NO₂, P(=O)(R⁴)₂, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, NR⁴, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy-oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden;
n ist bei jedem Auftreten gleich oder verschieden 0 oder 1, wobei mindestens zwei Indices n gleich 1 sein müssen; für n=0 ist an der entsprechenden Position eine Gruppe R² gebunden;
wobei die Verbindung keine Carbazolgruppe umfasst; und
wobei mindestens eine Gruppe Ar* in der Verbindung enthalten sein muss, welche eine elektronenreiche Heteroarylgruppe mit 5 bis 18 aromatischen Ringatomen oder ein aromatisches Ringsystem mit 12 bis 24 aromatischen Ringatomen darstellt.

2. Verbindung nach Anspruch 1, **dadurch gekennzeichnet, dass**
a) die Gruppe Ar* der folgenden Formel (H) entspricht 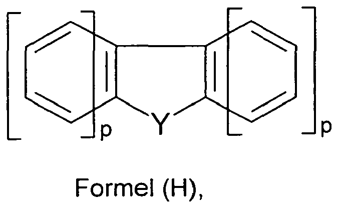 wobei
Y ausgewählt ist aus NR², PR², O und S; und
p bei jedem Auftreten gleich 0 oder 1 ist, wobei für p=0 an den betreffenden Positionen Reste R² gebunden sind, und wobei für Y=NR² nicht beide Indices p gleich 1 sein dürfen;
die Gruppe an allen freien Positionen mit Resten R² substituiert ist, und
die Gruppe R² wie in Anspruch 1 definiert ist, und
die Gruppe an einer beliebigen Position mit der Gruppe L verbunden sein kann, wobei die Bindung auch an die Stelle der Bindung NR² oder PR² treten kann; oder
b) die Gruppe Ar* ein aromatisches Ringsystem enthaltend 6 bis 24 aromatische Ringatome darstellt, welches mit einem oder mehreren Resten R² substituiert sein kann.

3. Verbindung nach Anspruch 1 oder 2, **dadurch gekennzeichnet, dass** die Gruppe L bei jedem Auftreten gleich oder verschieden eine Einfachbindung, Si(R²)₂, O, S, eine Alkylengruppe mit 1 bis 10 C-Atomen oder eine Alkenylen- oder Alkinylengruppe mit 2 bis 10 C-Atomen darstellt, wobei bei den genannten Gruppen eine oder mehrere CH₂-Gruppen durch Si(R²)₂, O oder S ersetzt sein können und wobei ein oder mehrere H-Atome in den genannten Gruppen durch D, F oder CN ersetzt sein können, oder ein aromatisches Ringsystem mit 6 bis 24 aromatischen Ringatomen darstellt, welches durch einen oder mehrere Reste R² substituiert sein kann.

4. Verbindung nach einem oder mehreren der Ansprüche 1 bis 3, **dadurch gekennzeichnet, dass** genau eine Gruppe L-Ar* an das Stickstoffatom des Grundgerüsts gemäß Formel (I) gebunden ist, und genau eine weitere Gruppe L-Ar* an den aromatischen Sechsring des Grundgerüsts gemäß Formel (I) gebunden ist.

5. Verbindung nach einem oder mehreren der Ansprüche 1 bis 4, **dadurch gekennzeichnet, dass** die Anbindungsposition der Gruppe L-Ar* an den aromatischen Sechsring des Grundgerüsts gemäß Formel (I) in der Position para oder meta zum Stickstoffatom vorliegt.

6. Verbindung nach einem oder mehreren der Ansprüche 1 bis 5, **dadurch gekennzeichnet, dass** X ausgewählt ist aus C(R¹)₂, O und S.

7. Verbindung nach einem oder mehreren der Ansprüche 1 bis 6, **dadurch gekennzeichnet, dass** maximal eine Gruppe Z pro aromatischem Sechsring des Grundgerüsts gemäß Formel (I) gleich N ist.

8. Verbindung nach einem oder mehreren der Ansprüche 1 bis 7, **dadurch gekennzeichnet, dass** für den Fall, dass X eine Gruppe C(R¹)₂ darstellt, die Gruppen R¹ der Gruppe C(R¹)₂ kein aromatisches Ringsystem und keine Arylgruppe darstellen.

9. Verbindung nach einem oder mehreren der Ansprüche 1 bis 8, **dadurch gekennzeichnet, dass** sie keine elektronenarme Heteroarylgruppe sowie keine Ketogruppe, keine Phosphoroxidgruppe und keine Schwefeloxidgruppe enthält.

10. Verfahren zur Herstellung einer Verbindung nach einem oder mehreren der Ansprüche 1 bis 9, **dadurch gekennzeichnet, dass** ausgehend von einem Dihydroacridin-Derivat eine oder mehrere übergangsmetallkatalysierte Kupplungsreaktionen durchgeführt werden, mit denen Aryl- oder Heteroarylgruppen als Substituenten eingeführt werden.

11. Oligomer, Polymer oder Dendrimer enthaltend eine oder mehrere Verbindungen nach einem oder mehreren der Ansprüche 1 bis 9, wobei die Bindung(en) zum Polymer, Oligomer oder Dendrimer an beliebigen, in Formel (I) mit R¹ oder R² substituierten Positionen lokalisiert sein können.

12. Formulierung enthaltend mindestens eine Verbindung nach einem oder mehreren der Ansprüche 1 bis 9 oder mindestens ein Polymer, Oligomer oder Dendrimer gemäß Anspruch 11 und mindestens ein Lösungsmittel.

13. Elektronische Vorrichtung, enthaltend mindestens eine Verbindung gemäß einem oder mehreren der Ansprüche 1 bis 9 oder mindestens ein Polymer, Oligomer oder Dendrimer gemäß Anspruch 11.

14. Elektronische Vorrichtung gemäß Anspruch 13, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

15. Elektronische Vorrichtung gemäß Anspruch 13, welche ausgewählt ist aus der Gruppe der organischen Elektrolumineszenz-vorrichtungen, **dadurch gekennzeichnet, dass** die Verbindung gemäß einem oder mehreren der Ansprüche 1 bis 9 oder das Polymer, Oligomer oder Dendrimer gemäß Anspruch 11 in einer oder mehreren der folgenden Funktionen eingesetzt wird:
- als Lochtransportmaterial in einer Lochtransport- oder Lochinjektionsschicht,
- als Matrixmaterial in einer emittierenden Schicht,
- als Elektronenblockiermaterial,
- als Excitonenblockiermaterial.

## Claims

1. Compound of a formula (I) 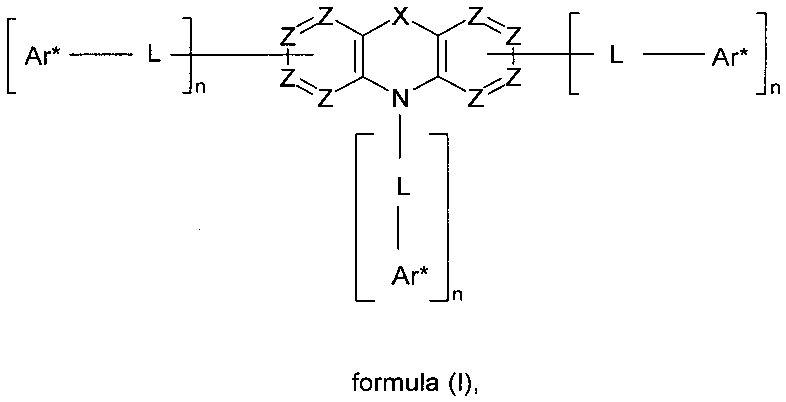 where the following applies to the symbols and indices occurring:
Ar* is on each occurrence, identically or differently, an aromatic ring system having 6 to 24 aromatic ring atoms or an electron-rich heteroaryl group having 5 to 18 aromatic ring atoms, each of which may be substituted by one or more radicals R²;
L is on each occurrence, identically or differently, a single bond, C=O, C=NR², Si(R²)₂, PR², P(=O)(R²), O, S, SO, SO₂, an alkylene group having 1 to 20 C atoms or an alkenylene or alkynylene group having 2 to 20 C atoms, where one or more CH₂ groups in the said groups may be replaced by C=O, C=NR², C=O-O, C=O-NR², Si(R²)₂, NR², P(=O)(R²), O, S, SO or SO₂ and where one or more H atoms in the above-mentioned groups may be replaced by D, F, Cl, Br, I, CN or NO₂, or an aromatic ring system having 6 to 24 aromatic ring atoms, which may be substituted by one or more radicals R², or any desired combination of 1, 2, 3, 4 or 5 identical or different groups selected from the above-mentioned groups;
X is a divalent group selected from C(R¹)₂, Si(R¹)₂, NR¹, PR¹, O and S;
Z is on each occurrence, identically or differently, CR² or N if Z carries no substituents, and is equal to C if Z carries a substituent;
R¹ is on each occurrence, identically or differently, H, D, F, Cl, Br, I, C(=O)R³, CN, Si(R³)₃, NO₂, P(=O)(R³)₂, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R³, where two or more nonaromatic radicals R¹ may be linked to one another and may form a ring;
R² is on each occurrence, identically or differently, H, D, F, Cl, Br, I, C(=O)R³, CN, Si(R³)₃, NO₂, P(=O)(R³)₂, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C=C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two or more radicals R² may be linked to one another and may form a ring;
R³ is on each occurrence, identically or differently, H, D, F, Cl, Br, I, C(=O)R⁴, CN, Si(R⁴)₃, NO₂, P(=O)(R⁴)₂, S(=O)R⁴, S(=O)₂R⁴, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C=C-, Si(R⁴)₂, C=O, C=S, C=NR⁴, -C(=O)O-, -C(=O)NR⁴-, NR⁴, P(=O)(R⁴), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring;
n is on each occurrence, identically or differently, 0 or 1, where at least two indices n must be equal to 1; for n=O, a group R² is bonded at the corresponding position;
where the compound does not contain a carbazole group; and
where at least one group Ar* which represents an electron-rich heteroaryl group having 5 to 18 aromatic ring atoms or an aromatic ring system having 12 to 24 aromatic ring atoms must be present in the compound.

2. Compound according to Claim 1, **characterised in that**
a) the group Ar* conforms to the following formula (H) 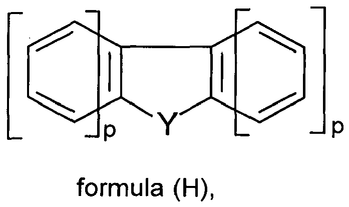 where
Y is selected from NR², PR², O and S; and
p is on each occurrence equal to 0 or 1, where, for p=0, radicals R² are bonded at the relevant positions, and where, for Y=NR², the two indices p cannot both be equal to 1;
the group is substituted by radicals R² at all free positions, and
the group R² is as defined in Claim 1, and
the group may be connected to the group L at any desired position, where the bonding may also occur at the site of the bond NR² or PR²; or
b) the group Ar* represents an aromatic ring system containing 6 to 24 aromatic ring atoms, which may be substituted by one or more radicals R².

3. Compound according to Claim 1 or 2, **characterised in that** the group L is on each occurrence, identically or differently, a single bond, Si(R²)₂, O, S, an alkylene group having 1 to 10 C atoms or an alkenylene or alkynylene group having 2 to 10 C atoms, where one or more CH₂ groups in the said groups may be replaced by Si(R²)₂, O or S and where one or more H atoms in the said groups may be replaced by D, F or CN, or an aromatic ring system having 6 to 24 aromatic ring atoms, which may be substituted by one or more radicals R².

4. Compound according to one or more of Claims 1 to 3, **characterised in that** precisely one group L-Ar* is bonded to the nitrogen atom of the basic structure of the formula (I), and precisely one further group L-Ar* is bonded to the aromatic six-membered ring of the basic structure of the formula (I).

5. Compound according to one or more of Claims 1 to 4, **characterised in that** the bonding position of the group L-Ar* to the aromatic six-membered ring of the basic structure of the formula (I) is in the para-or meta-position to the nitrogen atom.

6. Compound according to one or more of Claims 1 to 5, **characterised in that** X is selected from C(R¹)₂, O and S.

7. Compound according to one or more of Claims 1 to 6, **characterised in that** a maximum of one group Z per aromatic six-membered ring of the basic structure of the formula (I) is equal to N.

8. Compound according to one or more of Claims 1 to 7, **characterised in that**, in the case where X represents a group C(R¹)₂, the groups R¹ of the group C(R¹)₂ do not represent an aromatic ring system and do not represent an aryl group.

9. Compound according to one or more of Claims 1 to 8, **characterised in that** it contains no electron-deficient heteroaryl group and no keto group, no phosphorus oxide group and no sulfur oxide group.

10. Process for the preparation of a compound according to one or more of Claims 1 to 9, **characterised in that** one or more transition metal-catalysed coupling reactions by means of which aryl or heteroaryl groups are introduced as substituents are carried out starting from a dihydroacridine derivative.

11. Oligomer, polymer or dendrimer containing one or more compounds according to one or more of Claims 1 to 9, where the bond(s) to the polymer, oligomer or dendrimer may be localised at any desired positions in formula (I) that are substituted by R¹ or R².

12. Formulation comprising at least one compound according to one or more of Claims 1 to 9 or at least one polymer, oligomer or dendrimer according to Claim 11 and at least one solvent.

13. Electronic device comprising at least one compound according to one or more of Claims 1 to 9 or at least one polymer, oligomer or dendrimer according to Claim 11.

14. Electronic device according to Claim 13, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

15. Electronic device according to Claim 13, which is selected from the group of organic electroluminescent devices, **characterised in that** the compound according to one or more of Claims 1 to 9 or the polymer, oligomer or dendrimer according to Claim 11 is employed in one or more of the following functions:
- as hole-transport material in a hole-transport or hole-injection layer,
- as matrix material in an emitting layer,
- as electron-blocking material,
- as exciton-blocking material.

## Revendications

1. Composé d'une formule (I) : 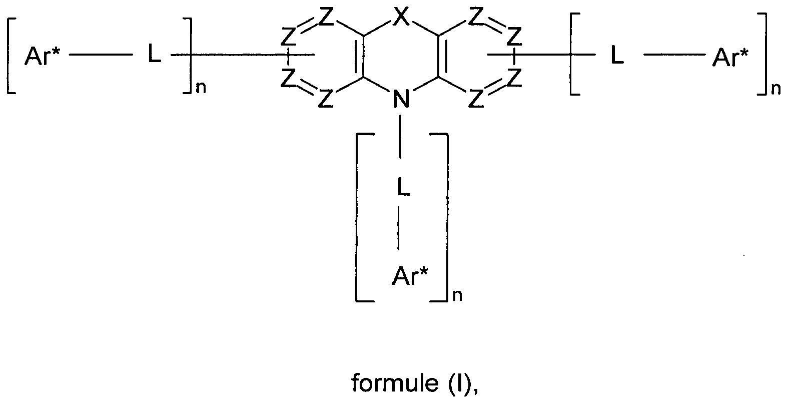 dans laquelle ce qui suit s'applique aux symboles et indices présents :
Ar* est, pour chaque occurrence, de manière identique ou différente, un système de cycle aromatique comportant de 6 à 24 atomes de cycle aromatique ou un groupe hétéroaryle riche en électrons comportant de 5 à 18 atomes de cycle aromatique, dont chacun peut être substitué par un ou plusieurs radical/radicaux R² ;
L est, pour chaque occurrence, de manière identique ou différente, une liaison simple, C=O, C=NR², Si(R²)₂, PR², P(=O)(R²), O, S, SO, SO₂, un groupe alkylène comportant de 1 à 20 atome(s) de C ou un groupe alkénylène ou alkynylène comportant de 2 à 20 atomes de C, où un ou plusieurs groupe(s) CH₂ parmi lesdits groupes peut/peuvent être remplacé(s) par C=O, C=NR², C=O-O, C=O-NR², Si(R²)₂, NR², P(=O)(R²), O, S, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique comportant de 6 à 24 atomes de cycle aromatique, lesquels peuvent être substitués par un ou plusieurs radical/radicaux R², ou une quelconque combinaison souhaitée de 1, 2, 3, 4 ou 5 groupes identiques ou différents choisis parmi les groupes mentionnés ci avant ;
X est un groupe divalent choisi parmi C(R¹)₂, Si(R¹)₂, NR¹, PR¹, O et S;
Z est, pour chaque occurrence, de manière identique ou différente, CR² ou N si Z n'est porteur d'aucun substituant, et est égal à C si Z est porteur d'un substituant ;
R¹ est, pour chaque occurrence, de manière identique ou différente, H, D, F, CI, Br, I, C(=O)R³, CN, Si(R³)₃, NO₂, P(=O)(R³)₂, S(=O)R³, S(=O)₂R³, un groupe alkyle, alcoxy ou thioalkyle en chaîne droite comportant de 1 à 20 atome(s) de C ou un groupe alkyle, alcoxy ou thioalkyle ramifié ou cyclique comportant de 3 à 20 atomes de C ou un groupe alkényle ou alkynyle comportant de 2 à 20 atomes de C, où les groupes mentionnés ci avant peuvent chacun être substitués par un ou plusieurs radical/radicaux R³ et où un ou plusieurs groupe(s) CH₂ dans les groupes mentionnés ci avant peut/peuvent être remplacé(s) par -R³C=CR³-, -C≡C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes mentionnés ci avant peut/ peuvent être remplacé(s) par D, F, Cl, Br, I, CN ou NO₂, ou un système de cycle aromatique comportant de 6 à 30 atomes de cycle aromatique, lesquels peuvent dans chaque cas être substitués par un ou plusieurs radical/radicaux R³, où deux radicaux non aromatiques ou plus R¹ peuvent être liés l'un à l'autre ou les uns aux autres et peuvent former un cycle ;
R² est, pour chaque occurrence, de manière identique ou différente, H, D, F, Cl, Br, I, C(=O)R³, CN, Si(R³)₃, NO₂, P(=O)(R³)₂, S(=O)R³, S(=O)₂R³, un groupe alkyle, alcoxy ou thioalkyle en chaîne droite comportant de 1 à 20 atome(s) de C ou un groupe alkyle, alcoxy ou thioalkyle ramifié ou cyclique comportant de 3 à 20 atomes de C ou un groupe alkényle ou alkynyle comportant de 2 à 20 atomes de C, où les groupes mentionnés ci avant peuvent chacun être substitués par un ou plusieurs radical/radicaux R³ et où un ou plusieurs groupe(s) CH₂ dans les groupes mentionnés ci avant peut/peuvent être remplacé(s) par -R³C=CR³-, -C≡C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes mentionnés ci avant peut/ peuvent être remplacé(s) par D, F, CI, Br, I, CN ou NO₂, ou un système de cycle aromatique ou hétéroaromatique comportant de 5 à 30 atomes de cycle aromatique, lesquels peuvent dans chaque cas être substitués par un ou plusieurs radical/radicaux R³, ou un groupe aryloxy ou hétéroaryloxy comportant de 5 à 30 atomes de cycle aromatique, lesquels peuvent être substitués par un ou plusieurs radical/radicaux R³, où deux ou plusieurs radicaux R² peuvent être liés l'un à l'autre ou les uns aux autres et peuvent former un cycle ;
R³ est, pour chaque occurrence, de manière identique ou différente, H, D, F, Cl, Br, I, C(=O)R⁴, CN, Si(R⁴)₃, NO₂, P(=O)(R⁴)₂, S(=O)R⁴, S(=O)₂R⁴, un groupe alkyle, alcoxy ou thioalkyle en chaîne droite comportant de 1 à 20 atome(s) de C ou un groupe alkyle, alcoxy ou thioalkyle ramifié ou cyclique comportant de 3 à 20 atomes de C ou un groupe alkényle ou alkynyle comportant de 2 à 20 atomes de C, où les groupes mentionnés ci avant peuvent chacun être substitués par un ou plusieurs radical/radicaux R⁴ et où un ou plusieurs groupes CH₂ dans les groupes mentionnés ci avant peut/peuvent être remplacé(s) par -R⁴C=CR⁴-, -C≡C-, Si(R⁴)₂, C=O, C=S, C=NR⁴, -C(=O)O-, -C(=O)NR⁴-, NR⁴, P(=O)(R⁴), - O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant de 5 à 30 atomes de cycle aromatique, lesquels peuvent dans chaque cas être substitués par un ou plusieurs radical/radicaux R⁴, ou un groupe aryloxy ou hétéroaryloxy comportant de 5 à 30 atomes de cycle aromatique, lesquels peuvent être substitués par un ou plusieurs radical/radicaux R⁴, où deux radicaux R³ ou plus peuvent être liés l'un à l'autre ou les uns aux autres et peuvent former un cycle ;
R⁴ est, pour chaque occurrence, de manière identique ou différente, H, D, F ou un radical organique aliphatique, aromatique ou hétéroaromatique comportant de 1 à 20 atome(s) de C, où, en outre, un ou plusieurs atome(s) de H peut/peuvent être remplacé(s) par D ou F ; deux substituants R⁴ ou plus peuvent ici être liés l'un à l'autre et peuvent former un cycle ;
n est, pour chaque occurrence, de manière identique ou différente, 0 ou 1, où au moins deux indices n doivent être égaux à 1 ; pour n=0, un groupe R² est lié au niveau de la position correspondante ;
où le composé ne contient pas un groupe carbazole ; et
où au moins un groupe Ar* qui représente un groupe hétéroaryle riche en électrons comportant de 5 à 18 atomes de cycle aromatique ou un système de cycle aromatique comportant de 12 à 24 atomes de cycle aromatique doit être présent dans le composé.

2. Composé selon la revendication 1, **caractérisé en ce que** :
a) le groupe Ar* est conforme à la formule (H) qui suit : 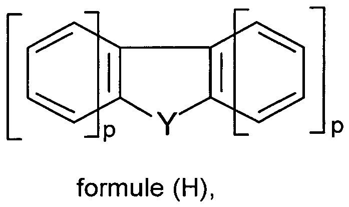 dans laquelle :
Y est choisi parmi NR², PR², O et S ; et
p est, pour chaque occurrence, égal à 0 ou 1, où, pour p=O, les radicaux R² sont liés au niveau des positions pertinentes, et où, pour Y=NR², les deux indices p ne peuvent pas être égaux à 1 ;
le groupe est substitué par des radicaux R² au niveau de toutes les positions libres, et
le groupe R² est comme défini selon la revendication 1, et
le groupe peut être connecté au groupe L au niveau de n'importe quelle position souhaitée, où la liaison peut également être présente au niveau du site de la liaison NR² ou PR² ; ou
b) le groupe Ar* représente un système de cycle aromatique contenant de 6 à 24 atomes de cycle aromatique, lesquels peuvent être substitués par un ou plusieurs radical/radicaux R².

3. Composé selon la revendication 1 ou 2, **caractérisé en ce que** le groupe L est, pour chaque occurrence, de manière identique ou différente, une liaison simple, Si(R²)₂, O, S, un groupe alkylène comportant de 1 à 10 atome(s) de C ou un groupe alkénylène ou un groupe alkynylène comportant de 2 à 10 atomes de C, où un ou plusieurs groupe(s) CH₂ dans lesdits groupes peut/peuvent être remplacé(s) par Si(R²)₂, O ou S et où un ou plusieurs atome(s) de H dans lesdits groupes peut/peuvent être remplacé(s) par D, F ou CN, ou un système de cycle aromatique comportant de 6 à 24 atomes de cycle aromatique, lesquels peuvent être substitués par un ou plusieurs radical/radicaux R².

4. Composé selon une ou plusieurs des revendications 1 à 3, **caractérisé en ce que** précisément un groupe L-Ar* est lié à un atome d'azote de la structure de base de la formule (I), et précisément un autre groupe L-Ar* est lié au cycle aromatique à six éléments de la structure de base de la formule (I).

5. Composé selon une ou plusieurs des revendications 1 à 4, **caractérisé en ce que** la position de liaison du groupe L-Ar* sur le cycle aromatique à six éléments de la structure de base de la formule (I) est dans la position para ou méta de l'atome d'azote.

6. Composé selon une ou plusieurs des revendications 1 à 5, **caractérisé en ce que** X est choisi parmi C(R¹)₂, O et S.

7. Composé selon une ou plusieurs des revendications 1 à 6, **caractérisé en ce qu'**un maximum d'un groupe Z par cycle aromatique à six éléments de la structure de base de la formule (I) est égal à N.

8. Composé selon une ou plusieurs des revendications 1 à 7, **caractérisé en ce que**, dans le cas où X représente un groupe C(R¹)₂, les groupes R¹ du groupe C(R¹)₂ ne représentent pas un système de cycle aromatique et ne représentent pas un groupe aryle.

9. Composé selon une ou plusieurs des revendications 1 à 8, **caractérisé en ce qu'**il ne contient aucun groupe hétéroaryle déficient en électrons et aucun groupe céto, aucun groupe oxyde de phosphore et aucun groupe oxyde de soufre.

10. Procédé pour la préparation d'un composé selon une ou plusieurs des revendications 1 à 9, **caractérisé en ce qu'**une ou plusieurs réactions de couplage catalysée(s) par métal de transition au moyen de laquelle ou desquelles des groupes aryle ou hétéroaryle sont introduits en tant que substituants est/sont mise(s) en oeuvre en partant d'un dérivé de dihydroacridine.

11. Oligomère, polymère ou dendrimère contenant un ou plusieurs composé(s) selon une ou plusieurs des revendications 1 à 9, où la/les liaison(s) sur le polymère, l'oligomère ou le dendrimère peut/peuvent être localisée(s) au niveau de n'importe quelles positions souhaitées dans la formule (I) qui sont substituées par R¹ ou R².

12. Formulation comprenant au moins un composé selon une ou plusieurs des revendications 1 à 9 ou au moins un polymère, un oligomère ou un dendrimère selon la revendication 11 et au moins un solvant.

13. Dispositif électronique comprenant au moins un composé selon une ou plusieurs des revendications 1 à 9 ou au moins un polymère, un oligomère ou un dendrimère selon la revendication 11.

14. Dispositif électronique selon la revendication 13, **caractérisé en ce qu'**il est choisi parmi des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC), des diodes laser organiques (O-laser) et des dispositifs électroluminescents organiques (OLED).

15. Dispositif électronique selon la revendication 13, lequel est choisi parmi le groupe des dispositifs électroluminescents organiques, **caractérisé en ce que** le composé selon une ou plusieurs des revendications 1 à 9 ou le polymère, l'oligomère ou le dendrimère selon la revendication 11 est utilisé au niveau d'une ou de plusieurs des fonctions qui suivent :
- en tant que matériau de transport de trous dans une couche de transport de trous ou d'injection de trous,
- en tant que matériau de matrice dans une couche d'émission,
- en tant que matériau de blocage d'électrons,
- en tant que matériau de blocage d'excitons.
